# EUROPEAN PATENT APPLICATION

(11) **EP 4 011 387 A1**
(43) Date of publication of application: **15.06.2022**
(21) Application number: 20213630.5
(22) Date of filing: 11.12.2020
(51) Int. Cl.: A61K 38/17

(54) **SUPERIOR NEUTRALIZATION OF SARS-COV-2 BY DEGLYCOSYLATED HUMAN ANGIOTENSIN CONVERTING ENZYME 2**

(71) Applicant: IMBA-Institut für Molekulare Biotechnologie GmbH, 1030 Wien (AT)
(72) Inventor: The designation of the inventor has not yet been filed
(74) Representative: Moré, Solveig Helga

(57) **Abstract**

The present invention relates to an ACE2 polypeptide lacking an N-linked glycosylation or comprising a truncated N-linked glycosylation, e.g., at an amino acid corresponding to Asn90 and/or Asn322 of SEQ ID NO: 1; its use in a treatment of a coronavirus infection and methods of manufacturing the ACE2 polypeptide.

## Description

The present invention relates to the field of glycosylation-modified ACE2 proteins.

### Background of the invention

The rapid spread of Severe Acute Respiratory Syndrome Coronavirus-2 (SARS-CoV-2), the causative pathogen of human coronavirus disease 2019 (COVID-19), has resulted in an unprecedented pandemic and worldwide health crisis. Similar to the beta-coronaviruses SARS-CoV and Middle Eastern Respiratory Syndrome (MERS)-CoV, SARS-CoV-2 is highly transmissible and can lead to lethal pneumonia and multi-organ failure. For infection and viral entry, the Spike surface protein of SARS-CoV-2 binds to angiotensin converting enzyme 2 (ACE2) on host cells (Wu et al., Nature 579, 265-269 (2020)). Recombinant soluble human ACE2 (rshACE2) has been shown to bind Spike (Wang et al., Cell 181, 894-904.e9 (2020)), can effectively neutralize SARS-CoV-2 infections (Monteil et al., Cell 181, 905-913.e7 (2020)).

The SARS-CoV-2 Spike protein is heavily glycosylated with both complex and oligo-mannosidic type N-glycans (Watanabe et al., Science 369, 330-333 (2020)), thereby shielding a large portion of the protein surface. Similarly, ACE2 is a glycoprotein. Recent studies investigated protein glycosylation in the context of the interaction between Spike and ACE2, with partially contradicting results (Zhao et al., Cell Host & Microbe 28, 586-601 (2020); Sun, et al., Engineering (2020) doi: 10.1016/j.eng.2020.07.014).

While many candidates for a therapy of COVID-19 are investigated, no drug has yet been approved. There is an ongoing need to find a drug that is suitable to treat COVID-19. Current approached need constant improvements. It is therefore a goal of the invention to provide a therapeutic medicament for the treatment of COVID-19 and to improve the prospects of possible candidates.

### Summary of the invention

The present invention provides an ACE2 polypeptide lacking an N-linked glycosylation of two or more sugar residues at an amino acid corresponding to Asn53, Asn90, Asn103, Asn322, Asn432, Asn546 and/or Asn690 of SEQ ID NO: 1; or comprising a truncated N-linked glycosylation an amino acid corresponding to Asn53, Asn90, Asn103, Asn322, Asn432, Asn546 and/or Asn690 of SEQ ID NO: 1, wherein the truncated N-linked glycosylation not larger than a -GlcNAc₂Man₃GlcNAc₂ structure.

The invention further provides an ACE2 polypeptide lacking an N-linked glycosylation of two or more sugar residues at an amino acid corresponding to Asn90 and/or Asn322 of SEQ ID NO: 1 or comprising a truncated N-linked glycosylation an amino acid corresponding to Asn90 and/or Asn322 of SEQ ID NO: 1, wherein the truncated N-linked glycosylation not larger than a -GlcNAc₂Man₃GlcNAc₂ structure, and comprising an N-linked glycosylation at one or more Asn residues corresponding to Asn53, Asn103, Asn432, Asn546 and/or Asn690 of SEQ ID NO: 1.

The invention further provides an ACE2 polypeptide lacking an N-linked glycosylation of two or more sugar residues at an amino acid corresponding to Asn53, Asn90, Asn103, Asn322, Asn432, Asn546 and/or Asn690 of SEQ ID NO: 1 or comprising a truncated N-linked glycosylation an amino acid corresponding to Asn53, Asn90, Asn103, Asn322, Asn432, Asn546 and/or Asn690 of SEQ ID NO: 1, further comprising at least one immunoglobulin domain.

Further provided is the use of any of the ACE2 polypeptides for use as a medicament. The invention also provides a pharmaceutical composition comprising an ACE2 polypeptide of the invention.

In particular, any of the ACE2 polypeptides can be for use in treating a coronavirus infection, in particular a SARS-CoV-2 or SARS-CoV-1 infection in a subject.

Also provided is a method to treat a coronavirus infection, in particular a SARS-CoV-2 or SARS-CoV-1 infection, in a subject comprising administering any of the ACE2 polypeptides.

Any ACE2 polypeptide can be used in method of manufacturing a medicament for the treatment of a coronavirus infection, in particular a SARS-CoV-1 or a SARS-CoV-2 infection.

Further provided is a composition comprising at least 700 µg ACE2 polypeptides, wherein at least 20% (molar-%) of the ACE2 polypeptides are the ACE2 polypeptides of the invention.

Also provided is a composition comprising at least 160 µg ACE2 polypeptides of the invention.

Further provided is an in vitro method of manufacturing an ACE2 polypeptide or a composition according to the invention, comprising expressing a nucleic acid encoding the ACE2 polypeptide in one or more cells.

Any of these aspects may be combined with each other and all detailed descriptions can be read on other aspects of the invention. E.g. any ACE2 polypeptides may be used in any method or in the kit. Descriptions of compositions or kits may be considered as preferred embodiments for the inventive uses of ACE2 polypeptides. The compositions may be administered to a subject, e.g. as a medicament. ACE2 polypeptides may be the product of the inventive methods of manufacture and descriptions of methods of manufacture may result in the inventive ACE2 polypeptides, compositions or kits.

### Detailed description of the invention

Infection and viral entry of SARS-CoV-2 crucially depends on the binding of its Spike protein to angiotensin converting enzyme 2 (ACE2) presented on host cells.

The Spike glycoprotein of SARS-CoV-2 is the protein responsible for allowing the SARS-CoV-2 virus to attach to the membrane of a host cell, the receptor binding domain ("RBD") of the spike protein of SARS-CoV-2 recognizes and e.g. attaches to ACE2 of host cells to use them as a mechanism of cell entry. As such, ACE2 is a receptor for the Spike glycoprotein of SARS-CoV-2. ACE2, in particular soluble versions thereof, can be administered to a patient for competitive binding of SARS-CoV-2.

ACE2 is a key metalloprotease of the Renin Angiotensin System (RAS), primarily existing as a membrane anchored zinc metalloprotease (WO 2004/000367). ACE2 is expressed in the vascular system as well as in most organs, but predominantly in the lungs, kidneys, liver, heart, intestine and testis. In a normal adult human lung, ACE2 is expressed primarily in alveolar epithelial type II cells, which can serve as a viral reservoir. These cells produce surfactant which reduces surface tension, thus preventing alveoli from collapsing, and hence are critical to the gas exchange function of the lung.

Glycosylation of both proteins is involved in this interaction. Engineering of ACE2 N-linked glycosylation by site-directed mutagenesis according to the invention resulted in enhanced binding affinities and improved virus neutralization. Importantly, removal of all N-glycans from clinical grade rshACE2 yields a superior SARS-CoV-2 decoy receptor for effective treatment for COVID-19 patients.

The invention provides an ACE2 polypeptide that has one or more modified N-linked glycans, in particular truncations or removals of at least one N-linked complex or hybrid glycan corresponding to the N-linked glycans at amino acids corresponding to Asn53, Asn90, Asn103, Asn322, Asn432, Asn546, Asn690 of SEQ ID NO: 1 or 2. In particular according to a central aspect, the invention provides an ACE2 polypeptide lacking an N-linked glycosylation of two or more sugar residues at an amino acid corresponding to Asn90 and/or Asn322 of SEQ ID NO: 1. These N-glycans may be specifically removed so that other N-glycans may remain. E.g. the ACE2 polypeptide may comprise an N-linked glycosylation at one or more Asn residues corresponding to Asn53, Asn103, Asn432, Asn546 and/or Asn690 of SEQ ID NO: 1. Of course the invention also provides ACE2 polypeptides with less, e.g. no N-linked glycan.

Wild-type ACE2 has several N-linked glycans, i.e at positions Asn53, Asn90, Asn103, Asn322, Asn432, Asn546, Asn690 of SEQ ID NO: 1 or 2. Asn690 is sometimes irregularly glycosylated and also according to the invention it may or may not be glycosylated.

N-linked glycans (also referred to as "N-glycans") are almost always attached to the nitrogen atom of an asparagine (Asn) side chain that is present as a part of Asn-X-Ser/Thr consensus sequence, where X is any amino acid except proline (Pro).

In case of wild-type ACE2 the consensus sequences are:
for the N-glycan at Asn53: Asn Ile Thr (amino acids 53-55);
for the N-glycan at Asn90: Asn Leu Thr (amino acids 90-92);
for the N-glycan at Asn103: Asn Gly Ser (amino acids 103-105);
for the N-glycan at Asn322: Asn Met Thr (amino acids 322-324);
for the N-glycan at Asn432: Asn Glu Thr (amino acids 432-434);
for the N-glycan at Asn546: Asn Ser Thr (amino acids 546-548);
for the N-glycan at Asn690: Asn Val Ser (amino acids 690-692). Amino acid positions are given as corresponding to human ACE2 of SEQ ID NO:1 or 2).

N-glycans come in a variety of glycosylation structures depending on the grade of processing in the golgi. Processing involves a stepwise conversion of high mannose glycans to complex glycans, which may have one of the following formulas: 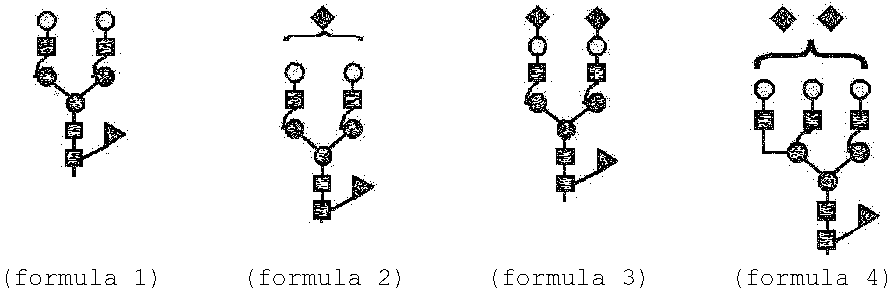 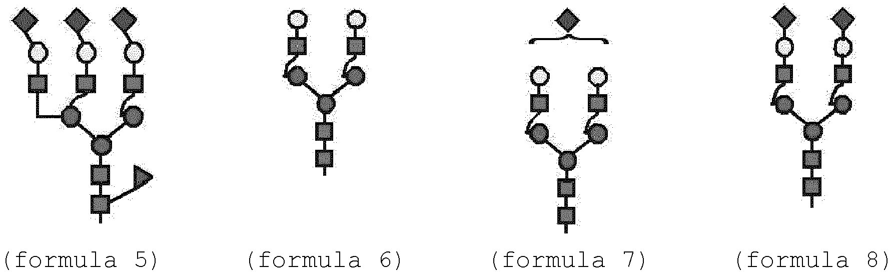 with

| | | | |
|---|---|---|---|
| G Ic N A c | ■ | F u c | ▲ |
| M a n | ● | X y l | ★ |
| G a I | ○ | N e u 5 A c | ◆ |

- see WO 2008/151347 (incorporated herein by reference).

N-linked glycans are usually composed of *N*-acetylglucosamine (GlcNAc), one being linked to the Asn of the consensus sequence (bottom GlcNAc in formulas 1-8), mannose (Man), galactose (Gal), fucose (Fuc) and N-acetylneuraminic acid (Neu5Ac), also termed sialic acid. Xylose (Xyl) is usually not present in mammalian cell produced ACE2 but can be artificially added with a xylosyl-transferase. These units of the glycan are collectively referred to as "sugar residues" herein. Variations in N-glycan structures are a result of incomplete and competing enzymatic reactions in the golgi.

The N-glycan of formula 3 is common and can be prevented from being formed or removed according to the invention in amino acids corresponding to Asn53, Asn90, Asn103, Asn322, Asn432, Asn546, Asn690 of SEQ ID NO: 1.

A truncation or removal of an N-linked glycan can be achieved by actively truncating or removing one or more of the N-linked glycans or by changing the amino acids sequence of ACE2 so that the ACE2 polypeptide is expressed with one or more missing N-linked glycan that would correspond to one or more of to Asn53, Asn90, Asn103, Asn322, Asn432, Asn546, Asn690 of SEQ ID NO: 1 or 2. For expression with a missing N-linked glycan the expression of the above consensus sequence may be altered, such as by removing one or more Asn corresponding to Asn53, Asn90, Asn103, Asn322, Asn432, Asn546, Asn690 of SEQ ID NO: 1 or 2; or by changing the subsequent amino acids in the consensus sequence Asn-X-Ser/Thr so that the Ser/Thr is removed or replaced by an amino acid that is not Ser and not Thr; or by replacing X with proline. In particular the ACE2 polypeptide may be expressed wherein one or more of the amino acids corresponding to Asn53, Asn90, Asn103, Asn322, Asn432, Asn546, Asn690 of SEQ ID NO: 1 or 2 is/are expressed as an amino acid selected from Ala, Cys, Asp, Glu, Phe, Gly, His, Ile, Lys, Leu, Met, Pro, Gln, Arg, Ser, Thr, Val, Trp, Tyr. Alternatively or in addition, the ACE2 polypeptide may be expressed wherein one or more of the amino acids corresponding to Thr55, Thr92, Ser105, Thr324, Thr434, Thr548, Ser692 of SEQ ID NO: 1 or 2 is/are expressed as an amino acid selected from Ala, Cys, Asp, Glu, Phe, Gly, His, Ile, Lys, Leu, Met, Asn, Pro, Gln, Arg, Val, Trp, Tyr. The invention also relates to the ACE2 polypeptides with these amino acid changes, as well as a nucleic acid encoding these ACE2 polypeptides.

In a preferred embodiment of the invention, the N-glycans at an amino acid corresponding to Asn90 and/or Asn322 are missing. In this preferred embodiment, the ACE2 polypeptide may contain a mutation in the N-linked glycosylation sequence corresponding to Asn90-Leu91-Thr92 of SEQ ID NO:1 and/or in the N-linked glycosylation sequence corresponding to Asn322-Met323-Thr324, wherein said mutation removes the N-linked glycosylation consensus sequence Asn-X-Ser/Thr, where X is any amino acid except proline, at amino acids corresponding to amino acids 90-92 and/or 322-324 of SEQ ID NO:1. Further N-glycans amino acids corresponding to Asn53, Asn103, Asn432, Asn546, Asn690 of SEQ ID NO: 1 may be present or missing, e.g. by similar mutations in the consensus sequence.

Preferred ACE2 polypeptides of the invention are ACE2-T92Q, ACE2-N332Q, ACE-T92Q-N322Q or fusion proteins thereof, e.g., with an immunoglobulin domain such as an Fc part.

An N-linked glycan may be removed enzymatically. Enzymatic removal may be with an endoglycosidase, e.g. endoglycosidase H or PNGase F. Enzymatic N-glycan removal may result in Asn deamidation, which may include the loss of a nitrogen and a hydrogen and the incorporation of an oxygen atom from water (Zheng et al., Virology 513 (2018) 65-74). This may create an aspartic acid residue in place of the asparagine residue on the protein. Other endoglycosidases hydrolyze the N,N'diacetylchitobiose moiety (between the two innermost GlcNAc residues) so that one GlcNAc residue remains on the protein/Asn residue. Example endoglycosidases are Endo H, Endo F1, Endo F2, Endo F3, Endo S, Endo S2, Endo D, Endo M.

The inventive glycan modifications "lacking an N-linked glycosylation of two or more sugar residues" takes account of the fact that enzymatic removal may leave a GlcNAc sugar residue on the Asn residue of the ACE2 polypeptide. The invention avoids longer N-glycans, such as complex or hybrid N-glycans, on the respective glycosylation sites. This GlcNAc is also the reducing end of the N-glycan. "Sugar residues" are any of the above-mentioned subunits of the N-glycan selected from GlcNAc, Man, Gal, Fuc, Neu5Ac and Xyl. One GlcNAc is not considered as an N-glycan that should be lacking in the inventive ACE2 polypeptide but of course in all instances this last GlcNAc may also be missing of the inventive ACE2 polypeptides.

According to the inventive glycan-modification of an ACE2 polypeptide a N-glycan according to any one of formulas 1-8 is avoided at an Asn corresponding to Asn53, Asn90, Asn103, Asn322, Asn432, Asn546, Asn690 of SEQ ID NO: 1 or 2. This can be done by modified expression of the ACE2 polypeptide, removal or truncation of one or more of these N-glycans.

Truncation of N-linked glycans can be done with a sialidase, a beta-galactosidase, an alpha-fucosidase, and/or beta-N-acetylglucosaminidase. A sialidase removes sialic acid residues from the N-glycan so that a galactose-terminated glycan remains. These can be processed further. A beta-galactosidase removes galactose residues from the N-glycan so that a GlcNAc-terminated N-glycan remains. Beta-N-acetylglucosaminidase cleaves terminal GlcNAc residues from N-glycans and can be used to convert the N-glycans to a small GlcNAc₂Man₃ structure, also termed MM structure due to the terminal mannose residues (non-reducing ends of the glycan). An alpha-fucosidase can be used to remove fucose residues to that a non-fucosylated N-glycan remains (Guthrie & Magnelli, BioProcess Int 2016, 14(2)). Preferably, the truncation is carried out with a beta-N-acetylglucosaminidase such as peptide-N4-(N-acetyl-beta-glucosaminyl)asparagine amidase F (PNGase F).

The invention also provides a method of truncating N-glycans on an ACE2 polypeptide comprising treatment of the N-glycan with a sialidase, beta-galactosidase, and/or beta-N-acetylglucosaminidase, optionally also an alpha-fucosidase. The invention also provides an ACE2 polypeptide obtainable (e.g., obtained) by contacting the ACE2 polypeptide with sialidase, beta-galactosidase, and/or beta-N-acetylglucosaminidase, preferably, a beta-N-acetylglucosaminidase such as peptide-N4-(N-acetyl-beta-glucosaminyl)asparagine amidase F (PNGase F).

In preferred embodiments, the truncation includes the removals of sialic acid residues and the removal of galactose residues. The resulting N-glycan may be a -GlcNAc₂Man₃GlcNAc₂ structure, wherein one or both terminal GlcNAc residues may be further removed, i.e. yielding a -GlcNAc₂Man₃GlcNAc structure or a-GlcNAc₂Man₃ structure, or an even smaller structure, with even fewer sugar residues ,such as 0, 1 or 2 Man residues. Accordingly, in preferred embodiments of the invention, the truncated N-linked glycosylation is not larger than a -GlcNAc₂Man₃GlcNAc₂ structure. "Larger" and "smaller" glycosylations means more or fewer sugar moieties, respectively. Preferably the truncation is found on an amino acid corresponding to Asn90 and/or Asn322 of SEQ ID NO: 1. The truncation may also or alternatively be on an Asn residue corresponding to one or more of Asn53, Asn103, Asn432, Asn546 and/or Asn690 of SEQ ID NO: 1. Truncated N-linked glycosylations may have the benefit of increased solubility than absent N-glycans on the ACE2 polypeptide while improving binding to Spike protein due to the removal of interfering sugar residues when compared to wild-type N-linked glycosylations.

Some N-glycans that have no or little interaction with the SARS-CoV-2 Spike protein may or may not be present on the inventive ACE2 polypeptide. These include N-glycans at an amino acid corresponding to Asn103 and/or Asn432 of SEQ ID NO: 1 or 2. In case of enzymatic de-glycosylation, these N-glycans may be missing, being removed or truncated. In other embodiments, e.g. in cases of site-specific mutagenesis, these N-glycans may be present, e.g. by maintaining the consensus sequence.

Likewise, a N-glycan at an amino acid corresponding to Asn690 of SEQ ID NO: 1 or 2 may be glycosylated or not.

Further N-glycans, in particular at an amino acid corresponding to Asn53 and/or Asn546, of SEQ ID NO: 1 or 2 may be removed or truncated to further improve ACE2 polypeptide binding to SARS-CoV-2 binding or they may be present to improved solubility of the ACE2 polypeptide.

Since some options exist, the invention also provides an ACE2 polypeptide lacking an N-linked glycosylation of two or more sugar residues at an amino acid corresponding to Asn53, Asn90, Asn103, Asn322, Asn432, Asn546 and/or Asn690 of SEQ ID NO: 1 or 2 or having a truncated N-linked glycosylation at an amino acid corresponding to Asn53, Asn90, Asn103, Asn322, Asn432, Asn546 and/or Asn690 of SEQ ID NO: 1 or 2. Truncations and lacking N-glycans can be combined, e.g. with one or more of these amino acid positions (Asn or modified as mentioned above) lacking an N-linked glycosylation and one or more of these amino acid positions comprising a truncated N-linked glycosylation. Preferably the ACE2 polypeptide lacks 2, 3, 4, 5, 6 or 7 of these N-linked glycosylations. Preferably the ACE2 polypeptide has 2, 3, 4, 5, 6 or 7 of these N-linked glycosylations truncated. In particular preferred is a combination of absent or truncated N-glycans selected from the group N-glycans at an amino acid corresponding to i) Asn90 and Asn322, ii) Asn90 and Asn546, iii) Asn322 and Asn546, iv) Asn53 and Asn90, v) Asn53 and Asn322, vi) Asn53, Asn90 and Asn322, vii) Asn90, Asn322 and Asn546, viii) Asn53, Asn90 and Asn546, ix) Asn53, Asn53, Asn322 and Asn546, x) Asn53, Asn90, Asn322 and Asn546, of SEQ ID NO: 1 or 2. The remaining N-glycans as described above may be glycosylated, wherein the N-glycan at an amino acid corresponding to Asn690 of SEQ ID NO: 1 or 2 may be optional, but is preferably present (or having a longer, wild-type glycosylation). Any of these ACE2 polypeptides is preferably for use as a medicament. Also preferred, these ACE2 polypeptides can be used in a method of manufacturing a medicament for the treatment of a coronavirus infection, in particular a SARS-CoV-1 or, preferably a SARS-CoV-2 infection. In particular preferred is the ACE2 polypeptide for use as medicament with the ACE2 polypeptide lacking N-linked glycosylation of at least two sugar residues at an amino acid corresponding to Asn90 and/or Asn322 of SEQ ID NO: 1.

The expression "amino acid corresponding to" Asn or amino acid number "of SEQ ID NO: 1" or "SEQ ID NO: 2" identifies the N-glycan sites according to the sequences of SEQ ID NO: 1 or 2. As is detailed herein, the ACE2 polypeptide can have modifications that may alter the amino acids or Asn position of the N-glycan site. In particular, truncations at the N-terminus are usual in order to increase solubility of the ACE2 polypeptide. Accordingly, the "amino acid corresponding to Asn53, Asn90, Asn103, Asn322, Asn432, Asn546 and/or Asn690 of SEQ ID NO: 1 or 2" may or may not be at positions 53, 90, 103, 322, 432, 546 and/or 690 of the inventive ACE2 polypeptide.

Many variants of ACE2 have been generated for therapeutic uses, including recombinant soluble ACE2 and shorter fragments suitable for glomerular filtration (WO 2008/151347, US 10,443,049 B2). In particular the C-terminus is suitable for large deletions while still maintaining activity of the enzyme.

Such variants, e.g. recombinant soluble ACE2 in deglycosylated form, e.g., as described above, may advantageously be used in the context of the invention. The ACE2 polypeptide of the invention still maintains the amino acids responsible for binding of Spike glycoprotein of SARS-CoV-2 as was investigated in several references (Walls et al., Cell (2020) 181(2): 281-292; Wan et al., J Virol (2020) doi:10.1128/JVI.00127-20; Wrapp et al., Science (2020) doi:10.1126/science.abb2507), so that it can act as molecular decoy to block virus entry.

The amino acid sequence of human recombinant ACE2 amino acids 1 to 740 is provided in SEQ ID NO: 1. SEQ ID NO: 2 provides the full-length amino acid sequence of human ACE2 with 805 amino acids in length. The full-length amino acid sequence is also provided in database UniProtKB, database entry Q9BYF1 as of 17 June 2020 (human ACE2). Amino acids 1-17 are the signal sequence, amino acids 19-740 form the extracellular domain, amino acids 741-761 are the transmembrane domain and amino acids 762-805 are the cytoplasmic domain. Amino acids 1 to 740 of SEQ ID NO:2 are identical to SEQ ID NO: 1. Corresponding amino acids and domains exist in other ACE2 polypeptides, including ACE2 from other mammals.

ACE2 polypeptides of the invention can be variants of naturally occurring ACE2 proteins. Such ACE2 variants may be used in methods and products of the invention. Changes which result in production of a chemically equivalent or chemically similar amino acid sequence are included within the scope of the invention. Variants of ACE2 may occur naturally, for example, by mutation, or may be made, for example, with polypeptide engineering techniques such as site directed mutagenesis, which are well known in the art for substitution of amino acids. For example, a hydrophobic residue, such as glycine can be substituted for another hydrophobic residue such as alanine. An alanine residue may be substituted with a more hydrophobic residue such as leucine, valine or isoleucine. A negatively charged amino acid such as aspartic acid may be substituted for glutamic acid. A positively charged amino acid such as lysine may be substituted for another positively charged amino acid such as arginine.

Therefore, the invention includes polypeptides having conservative changes or substitutions in amino acid sequences. Conservative amino acid substitutions insert one or more amino acids, which have similar chemical properties as the replaced amino acids. The invention includes sequences where conservative amino acid substitutions are made that do not destroy enzymatic activity and/or binding to Spike glycoprotein of SARS-CoV-2. Amino acids 147-555 of SEQ ID NO: 1 or 2 are considered important for catalytic activity and should preferably be retained with a high degree in the ACE2 polypeptide of the invention. The ACE2 polypeptide of the invention preferably comprises a sequence with a sequence identity of at least 90%, preferably at least 95& or at least 98% or at least 99%, to amino acids 147-555 of SEQ ID NO: 1 or 2. Other amino acid changes may lead to a loss of enzymatic activity, however maintaining or even increasing enzymatic activity and/or binding to Spike glycoprotein of SARS-CoV-2 is preferred.

Identity is calculated according to methods known in the art. Sequence identity is most preferably assessed by the BLAST version 2.1 program advanced search (parameters as above). BLAST is a series of programs that are available online at blast.ncbi.nlm.nih.gov/. The BLAST search may be set to default parameters (i.e. Matrix BLOSUM62; Gap existence cost 11; Per residue gap cost 1; Lambda ratio 0.85 default). References to BLAST searches are: Altschul et al., J. Mol. Biol. (1990) 215: 403-410; Gish & States, Nature Genet. (1993) 3: 266-272; Madden et al., Meth. Enzymol. (1996) 266: 131-141; Altschul et al. Nucleic Acids Res. (1997) 25: 3389-3402.

"Conservative amino acid substitutions" are those substitutions that are predicted to interfere least with the properties of the reference polypeptide. In other words, conservative amino acid substitutions substantially conserve the structure and the function of the reference protein. The following Table 1 provides a list of exemplary conservative amino acid substitutions:

| Original Residue | Conservative Substitution |
|---|---|
| Ala | Gly, Ser |
| Arg | His, Lys |
| Asn | Asp, Gin, His |
| Asp | Asn, Glu |
| Cys | Ala, Ser |
| Gln | Asn, Glu, His |
| Glu | Asp, Gln, His |
| Gly | Ala |
| His | Asn, Arg, Gin, Glu |
| Ile | Leu, Val |
| Leu | Ile, Val |
| Lys | Arg, Gln, Glu |
| Met | Leu, Ile |
| Phe | His, Met, Leu, Trp, Tyr |
| Ser | Cys, Thr |
| Thr | Ser, Val |
| Trp | Phe, Tyr |
| Tyr | His, Phe, Trp |
| Val | Ile, Leu, Thr |

Conservative amino acid substitutions generally maintain one or more of: (a) the structure of the polypeptide backbone in the area of the substitution, for example, as a beta sheet or alpha helical conformation, (b) the charge or hydrophobicity of the molecule at the site of the substitution, and/or (c) the bulk of the side chain.

Preferably about 1, 2, 3, 4, 5, 6 to 10, 11 to 25, 26 to 50 or 51 to 100, or 101 to 250 amino acids of SEQ ID NO: 1 or SEQ ID NO: 2 are modified or deleted. The invention includes polypeptides with mutations that cause an amino acid change in a portion of the polypeptide not involved in providing activity of SARS-Cov-2 binding or an amino acid change in a portion of the polypeptide involved in providing activity or SARS-Cov-2 binding so that the mutation increases or decreases the activity or SARS-Cov-2 binding of the polypeptide. For example, it is possible to increase SARS-Cov-2 spike protein binding of the ACE2 polypeptide according to the known interactions (Walls et al., Cell (2020) 181(2): 281-292; Wan et al., J Virol (2020) doi:10.1128/JVI.00127-20; Wrapp et al., Science (2020) doi:10.1126/science.abb2507). Improvement can be in comparison to an unmodified ACE2 of SEQ ID NO: 1. Amino acid changes to improve SARS-Cov-2 spike protein binding of ACE2 polypeptide can e.g. be to the receptor binding domain of the spike protein. Amino acids of ACE2 polypeptide are e.g. K31, E35, D38, M82 and K353 of SEQ ID NO: 1 or 2. These amino acids and adjacent amino acids or regions including amino acids 25 to 45, 75 to 90 or 345 to 360 corresponding to SEQ ID NO: 1 or 2 may be changed as compared to SEQ ID NO: 1 or 2 in an ACE2 polypeptide of the invention to alter spike protein binding.

In preferred embodiment, the ACE2 polypeptide comprises amino acids 19 to 600 of SEQ ID NO: 1. In particular preferments, the ACE2 polypeptide consists of or comprises amino acids 19 to 605 or amino acids 19 to 619, each of SEQ ID NO: 1, including embodiments of the ACE2 polypeptide comprising amino acids 1 to 605 or amino acids 1 to 619, each of SEQ ID NO: 1. Such ACE2 polypeptides, being fragments of native human ACE2 but retaining its activity are disclosed in US 10,443,049. In preferred embodiments the ACE2 polypeptide comprises or consists of an amino acid sequence with at least 70%, preferably at least 80%, at least 90%, at least 95%, at least 98% or at least 99%, sequence identity to amino acids 19 to 619 of SEQ ID NO: 1 or with amino acids 19 to 605 of SEQ ID NO: 1.

Preferably, the ACE2 polypeptide consists of or comprises amino acids 18 to 740 of SEQ ID NO: 1. Such ACE2 polypeptides are disclosed in WO 2008/151347 and in WO 2014/108530 (both incorporated herein by reference) and are preferred embodiments of the ACE2 polypeptide used according to the invention. In preferred embodiments the ACE2 polypeptide comprises or consists of an amino acid sequence with at least 70%, preferably at least 80%, at least 90%, at least 95%, at least 98% or at least 99%, sequence identity to SEQ ID NO: 1 or with amino acids 18 to 740 of SEQ ID NO: 1.

Preferably, a serine (or C-terminal amino acid) of the ACE2 polypeptide corresponding to Ser740 of SEQ ID NO: 1 (for example the C-terminal end) is O-glycosylated or is not O-glycosylated.

Polypeptides comprising one or more d-amino acids are contemplated within the invention. Also contemplated are polypeptides where one or more amino acids are acetylated at the N-terminus. Those with skill in the art recognize that a variety of techniques are available for constructing polypeptide mimetics with the same or similar desired compound activity as the corresponding polypeptide compound of the invention but with more favourable activity than the polypeptide with respect to solubility, stability, and/or susceptibility to hydrolysis and proteolysis.

Preferably, the ACE2 polypeptide is soluble ACE2 or a fusion protein thereof. "Soluble" refers to solubility in water, especially under physiological conditions, in the sense that the ACE2 polypeptide is not adhered to a cellular surface. Soluble ACE2 polypeptides thus lack an anchor region that would bind it to a cell membrane, such as a functional transmembrane domain as found in native ACE2. In particular, the transmembrane domain corresponding to amino acids 741-761 of SEQ ID NO: 2 should be missing in a soluble ACE2 polypeptide.

PEGylation is one of the preferred modifications of the ACE2 polypeptide but any fusion or modification as known in the art for pharmaceutical proteins can be used according to the invention. Such fusions or modifications are disclosed in Strohl et al., BioDrugs (2015) 29:215-239 (incorporated herein by reference) and include immunoglobulin domain fusions, Fc fusion proteins, scFva fusion, fusion to human serum albumin, fusion to human transferrin, fusion to carboxy-terminal peptide, and other polypeptide fusions, XTENylation, rPEG, PASylation, ELPylation, HAPylation, GLK fusion, CTP fusion. Such fusions and modifications can adjust the ACE2 polypeptide to a desirable pharmacokinetic profile, in particular increase of the half-life. An Fc fusion is preferably to an Fc of IgG, IgM, IgD, or IgA or a part thereof, such as a CH1, CH2 or CH3 domain, or FcRn. A CH3 domain is preferred. It may or may not include the C-terminus of the Fc part. IG is preferably human IgG₁, IgG₂, and IgG₄. Modifications, amino acid changes, selected glycosylation patters and fusions can protect the ACE2 polypeptide from proteolytic degradation, e.g. reduce proteolytic degradation as compared to unmodified ACE2, such as according to SEQ ID NO: 1 without modifications or fusions. Proteolytic degradation may from or in human serum.

The invention in a preferred embodiment includes hybrids and polypeptides of ACE2 polypeptides, for example where an amino acid sequence is combined with a second sequence. A possibility is a fusion with an antibody portion, in particular an immunoglobulin domain, such as a Fc fragment or a CH3 domain of a Fc fragment (US 10,443,049 B2). Accordingly, the invention provides an ACE2 polypeptide lacking an N-linked glycosylation of two or more sugar residues at an amino acid corresponding to Asn53, Asn90, Asn103, Asn322, Asn432, Asn546 and/or Asn690 of SEQ ID NO: 1, further comprising at least one immunoglobulin domain. Preferably, the ACE2 polypeptide comprises a heavy chain immunoglobulin domain, preferably an antibody CH1, CH2 or CH3 domain, more preferred comprising an Fc fragment.
An ACE2 polypeptide of the invention may further comprise a tag suitable for purification, such as a His-tag, e.g., an N-terminal His-Tag. For example, the ACE2 polypeptide of the invention comprises an N-terminal His-tag and at least one immunoglobulin domain, such as an Fc domain, preferably, a human Fc domain.

The ACE2 polypeptide is preferably catalytically active in hydrolysing angiotensin II to angiotensin-(1-7) and/or in hydrolysing angiotensin I to angiotensin-(1-9) (Vickers et al., J Biol Chem (2002) 277(17): 14838-14843). Preferably, the catalytic activity of the ACE2 polypeptide or preparation, ccat, is at least 4 s⁻¹, preferably at least 5 s⁻¹, particularly preferably at least 6 s⁻¹, highly preferably at least 7 s⁻¹, and most preferably at least 7.6 s⁻¹ with respect to the Ang 1-7 (angiotensin 1-7) conversion. Ang 1-7 is formed from Ang II (angiotensin II) by means of ACE2. The conversion can be tested in a simple manner, as described in WO 2008/151347 (incorporated herein by reference). This conversion or the catalytic activity of the ACE2 polypeptide can also be extrapolated from other assay data. The activity can, for example, be measured as described in WO 2008/046125 A (incorporated herein by reference).

The invention also provides an *in vitro* method of manufacturing an ACE2 polypeptide of the invention or a comprising the ACE2 polypeptides, comprising expressing a nucleic acid encoding the ACE2 polypeptide in one or more cells. The cells are preferably mammalian cells. The cells can also be bacterial cells, wherein the polypeptide will not be glycosylated. The method may further comprise a removal of one or more N-linked glycans, or the expression with a modified N-linked glycosylation site consensus sequence as described above. The method may further comprise purifying and/or isolating the ACE2 polypeptide. The method can further comprise enriching the ACE2 polypeptide to a concentration as desired for a composition of the ACE2 polypeptide.

The invention further provides any of the inventive ACE2 polypeptides for use in treating or preventing a coronavirus infection in a subject, preferably, for use in treatment. The coronavirus may be, e.g., SARS-CoV-2 or SARS-CoV-1 or MERS. The treatment may be a treatment of COVID-19. Related thereto, the invention provides a method of treating or preventing a coronavirus infection, e.g. COVID-19. The invention also relates to the method of manufacturing a medicament for the treatment of a coronavirus infection, e.g. COVID-19. All these aspects are interrelated and described collectively with the same preferred embodiments. Treatment of the infection may comprise treatment of at least one symptom of the infection. Preferably, the virus load of the treated subject is also reduced or even eliminated by the treatment. Prevention of an infection may be reduction of the incidence of infection or reduction of the likelihood of infection with said coronavirus. In a preferred embodiment, the ACE2 polypeptide of the invention is for use in preventing COVID-19 in a subject infected with SARS-CoV-2, in particular, for use in preventing serious complications, e.g., pulmonary symptoms of COVID-19 such as acute respiratory distress syndrome (ARDS) in the context of COVID-19, and/or for increasing oxygen saturation in a subject with COVID-19. For example, a subject diagnosed to be infected with SARS-CoV-2 can be treated with the ACE2 polypeptide of the invention after onset of symptoms, but before mechanical ventilation is indicated, or instead of mechanical ventilation or while the subject is treated by mechanical ventilation. Treatment of the subject according to the invention may also reduce the likelihood that said subject transmits the infection to another subject.

The inventive ACE2 polypeptide can be administered to a subject, preferably for use in treating a coronavirus infection. In particular an infection with a coronavirus that comprises a spike protein that binds to ACE2. Binding can be a binding affinity of the ACE2 polypeptide to monomeric RBD of SARS-CoV-2 Spike protein with an equilibrium affinity constant (*K*_{D}) of 500 nM or less, preferably 100 nM or less. In particular, the inventive glycan-optimization may lead to a binding affinity of 20 nM or less. Measurements can be at a pH of 7.4 and a temperature of 36°C. Binding affinity is preferably determined with an immobilized ACE2 polypeptide as e.g. described in Chan et al., Science 369, 1261-1265 (2020).

The ACE2 polypeptide of the invention may also be for use in regulating blood pressure, e.g., in reducing high blood pressure. It may be used for treating pulmonary arterial hypertension (PAH) and/or acute respiratory distress syndrome (ARDS). It may also be for use in protecting at least one organ such as the heart, kidney, blood vessels and lung via enzymatic degradation of angiotensin II in a subject in need thereof.

The treatment or prevention includes administering the ACE2 polypeptide of the invention to a subject in need thereof, e.g. a patient suffering from COVID-19 or a subject having a coronavirus infection. The ACE2 polypeptide for use in the treatment may be any one as described above, e.g. a ACE2 polypeptide that lacks an N-linked glycosylation of two or more sugar residues at an amino acid corresponding to Asn53, Asn90, Asn103, Asn322, Asn432, Asn546 and/or Asn690 of SEQ ID NO: 1, or comprises a truncated N-linked glycosylation at at least one, preferably two of these sites. Preferably it is a ACE2 polypeptide that lacks an N-linked glycosylation of two or more sugar residues at amino acids corresponding to Asn53, Asn90, Asn103, Asn322, Asn432 and Asn546 of SEQ ID NO: 1. Generally, an ACE2 polypeptide having a non-wildtype N-linked glycosylation pattern, wherein at least one, preferably, two, e.g., three, four, five or six or all N-linked glycosylations at an amino acid corresponding to Asn53, Asn90, Asn103, Asn322, Asn432, Asn546 and/or Asn690 of SEQ ID NO: 1 are lacking or truncated may be used in the treatment of the coronavirus infection. It may also be a deglycosylated ACE2 polypeptide, e.g., obtainable by treatment with a suitable enzyme, as described herein.

Surprisingly, the inventors found that use of an enzymatically deglycosylated protein, e.g., contacted with PNGaseF, is preferred, because it leads to a particularly advantageous form wherein some glycosylation sites are fully deglycosylated and some are partly glycosylated, as shown herein. It also maintains stability and/or inhibits aggregation of the protein during production, which may reduce yields upon recombinant production of ACE2 polypeptides mutated to lack glycosylation sites.

The ACE2 polypeptide is preferably for administration at a dose of 10 µg/kg to 1500 µg/kg daily. In particular preferred embodiments, the daily dose is about 400 µg/kg, in other embodiments, the daily dose is about 200 µg/kg. ACE2 is also investigated as a sole active ingredient and the daily dose of about 400 µg/kg may be used for such aspects. Given that recombinant soluble ACE2 is well tolerated, a broader dose range is possible. Accordingly, the invention provides for a daily dose of 10 µg/kg to 100 µg/kg, 100 µg/kg to 200 µg/kg, 200 µg/kg to 300 µg/kg, 300 µg/kg to 400 µg/kg, 400 µg/kg to 500 µg/kg, 500 µg/kg to 600 µg/kg, 700 µg/kg to 800 µg/kg, 800 µg/kg to 1000 µg/kg, 1000 µg/kg to 1500 µg/kg and any combination of these ranges, such as 200 µg/kg to 600 µg/kg, or 10 µg/kg to 300 µg/kg. µg/kg refers to the amount of ACE2 polypeptide in µg per kg of the patient's body weight. A skilled artisan (e.g. a physician or veterinarian) may reduce or increase dosage in accordance with these or other conditions or requirements.

Variants of ACE2 have been described as ACE2 polypeptides suitable for the present invention. Despite molecular weight differences of different ACE2 polypeptides, the above amounts refer to any ACE2 polypeptide, given that differences in molar concentration for a given mass amount are minor.

The treatment may be with a pharmaceutical composition. The invention also provides a composition comprising at least 700 µg ACE2 polypeptides, wherein at least 20%, (molar-%) of the ACE2 polypeptides are the ACE2 polypeptides according to the invention. Preferably the proportion of ACE2 polypeptides to all ACE2 polypeptides in the compositions is higher, such as at least 30%, at least 40%, at least 50% at least 60%, at least 70% at least 80% and in particular preferred at least 90% or even 100% (all molar-%). In particular, the amount of ACE2 polypeptides lacking an N-linked glycosylation at an amino acid corresponding to Asn90 and/or Asn322 of SEQ ID NO: 1 is preferably at least at least 30%, at least 40%, at least 50% at least 60%, at least 70% at least 80% and in particular preferred at least 90% or even 100% (all molar-%). Also provided is a composition comprising at least 160 µg ACE2 polypeptides according to the invention, e.g. of the ACE2 polypeptides lacking an N-linked glycosylation at an amino acid corresponding to Asn90 and/or Asn322 of SEQ ID NO: 1 or any other ACE2 polypeptides as described above. The ACE2 polypeptide may also provided in a kit. A kit may comprise one or more, e.g. 2, 3, 4, 5, 6, 7, 8 ACE2 polypeptide compositions in separate containers, such as vials, flasks, syringes or bags.

The inventive kits and pharmaceutical compositions preferably contain dosage forms for these daily doses in a container for a 70 kg subject. E.g. the kit or pharmaceutical composition may comprise the ACE2 polypeptide in an amount of 700 µg to 105 mg per container, more preferably 700 µg to 1 mg, 1 mg to 5 mg, 5 mg to 10 mg, 10 mg to 20 mg, 20 mg to 30 mg, 30 mg to 40 mg, 40 mg to 50 mg, 60 mg to 70 mg, 80 mg to 90 mg, 90 mg to 105 mg, each per container. Any combination of these ranges is possible, such as 10 mg to 40 mg, or 1 mg to 20 mg for lower doses than 400 µg/kg as mentioned above, given the efficacy of the inventive combination therapy.

The daily dose may be administered once per day, or as split doses more than once per day, wherein the daily dose is divided by the number of administrations to the patient on a day. The administration may be 2x per day, 3x per day or more often. It is also possible to administer with intermittent administration-free days, such as every 2^{nd} day (with a dose twice the daily dose). In case of non-daily administrations, the daily dose is adjusted so that the daily dose is achieved on average per day over the treatment time span. The treatment time span may be 1, day, 2 days, 3 days, 4 days, 5 days, 6 days, 7 days, 8 days, 9 days, 10 days, 11 days, 12 days, 13 days, 14 days, 15 days or more, such as up to 30 days or up to 40 days or even more. Any ranges in between these treatment times is possible.

In some embodiments, the ACE2 polypeptide is administered to the subject at about 0.4 mg/kg through intravenous injection twice a day up to 7 days.

In a preferred embodiment, the ACE2 polypeptide of the invention is for inhalation and/or intrapulmonary or intranasal administration. For example, inhalators or sprays may be used. The ACE2 polypeptide of the invention can also be administered to a patient by a combination of intravenous application and inhalation and/or intrapulmonary and/or intranasal administration.

Although ACE2 polypeptides based on human ACE2, e.g., having at least 70%, at least 80%, at least 90%, at least 95%, at least 99% or 100% sequence identity to human ACE2 (e.g., SEQ ID NOs 1 and 2) is preferred for most therapeutic applications, ACE2 from other mammals, for example cat, mink, ferret, dog, rat, hamster, pig, primates or cattle, can also be used. ACE2 is a universal enzyme in all mammals with the Ang II substrate which is identical in the various species. Hence, in principle it can also be used in other organisms. Thus, the ACE2 polypeptide according to the invention can be used regardless of the origin of the ACE2, for example from humans, cat, mink, ferret, dog, rats, hamsters, pigs, primates or cattle. However, in preferred embodiments, the origin of the ACE2 and the organism (subject or patient) to be treated is the same.

As used herein, the term "subject" may be used interchangeably with the term "patient" or "individual" and may include an "animal" and in particular a "mammal", that can be treated according to the invention. Mammalian subjects may include humans and non-human primates, domestic animals, farm animals, and companion animals such as dogs, cats, guinea pigs, rabbits, rats, mice, horses, cattle, cows, and the like. Preferably, the patient to be treated by the inventive method and uses of the invention is a human, preferably a human adult of the age of 18 years or more.

In accordance with the invention, a pharmaceutical composition or medicine comprising the ACE2 polypeptide can be provided. The pharmaceutical composition may be in a container, such as a vial, flask, syringe, bag, and/or in kit. An inhalator or spraying device comprising the composition is also provided. Such compositions may be pharmaceutically acceptable salts themselves, with additional buffers, tonicity components or pharmaceutically acceptable carriers. Pharmaceutical carrier substances serve to improve the compatibility of the composition and provide better solubility as well as better bioavailability of the active ingredients. Examples are emulsifiers, thickeners, redox components, starches, alcoholic solutions, polyethylene glycol and lipids. Selection of a suitable pharmaceutical carrier is highly dependent on the administration route. For oral administration, liquid or solid carriers may be used; for injections, liquid final compositions are required.

Preferably, the ACE2 polypeptide is provided in a composition comprising buffers or tonic substances. The buffer can adjust the pH of the medicine to the physiological conditions and further, can reduce or buffer variations in pH. An example is a phosphate buffer. Tonic substances can adjust the osmolarity and may include ionic substances, such as inorganic salts, for example NaCl or KCl, or non-ionic substances such as glycerin or carbohydrates. The invention provides a pharmaceutical composition comprising an ACE2 polypeptide of the invention, such as a deglycosylated ACE2 polypeptide of the invention as described herein, and further comprising an aqueous solution, e.g., water for injection or a buffer, e.g., a physiological buffer such as PBS. The physiological buffer may have a pH of 6.5-8, preferably, 7-7.5, e.g., 7.2-7.4. The pharmaceutical composition may also be lyophilized. It may be dissolved in water for injection or a physiological buffer before administration. The ACE2 polypeptide of the invention may alternatively be administered as a powder.

Preferably, the composition for use in accordance with the invention is suitably prepared for systemic, topical, oral, intrapulmonary or intranasal administration or as an inhaled preparation. Such administration routes are preferred embodiments of the inventive methods. These forms of administration for the composition of the present invention allow fast, uncomplicated take-up. When the ACE2 polypeptide is intended for oral administration, it is preferably provided in a formulation which is resistant to stomach acid or it is encapsulated. For oral administration, solid or liquid medicines can be taken directly or dissolved or diluted, for example. The pharmaceutical composition or kit for use in accordance with the invention is preferably produced for intravenous, intra-arterial, intramuscular, intravascular, intraperitoneal or subcutaneous administration. Injections or transfusions, for example, are suitable for this purpose. Administration directly into the bloodstream has the advantage that the active ingredient of the medicine can be distributed through the entire body and the target tissue, such as lungs, heart, kidney, intestine or liver, is reached quickly.

The composition may be pharmaceutically acceptable. The term "pharmaceutically acceptable" indicates that the designated carrier, vehicle, diluent, excipient(s), and/or salt is generally chemically and/or physically compatible with the other ingredients comprising the formulation, and physiologically compatible with the recipient thereof. In some embodiments, compounds, materials, carriers, compositions, and/or dosage forms that are pharmaceutically acceptable refer to those approved by a regulatory agency (such as U.S. Food and Drug Administration, National Medicine or European Medicines Agency) or listed in generally recognized pharmacopoeia (such as U.S. Pharmacopoeia, China Pharmacopoeia or European Pharmacopoeia) for use in animals, and more particularly in humans.

Pharmaceutical acceptable carriers for use in the pharmaceutical compositions disclosed herein may include, for example, pharmaceutically acceptable liquid, gel, or solid carriers, aqueous vehicles, non-aqueous vehicles, antimicrobial agents, isotonic agents, buffers, tonicity-adjusting agents, antioxidants, anesthetics, suspending/dispending agents, sequestering or chelating agents, diluents, adjuvants, excipients, or non-toxic auxiliary substances, other components known in the art, or various combinations thereof. Suitable carriers and auxiliary components may include, for example, fillers, binders, disintegrants, buffers, preservatives, lubricants, flavorings, thickeners, coloring agents, emulsifiers or stabilizers such as sugars and cyclodextrins. In some embodiments, the suitable buffers may include, for example, a phosphate buffer or a MES (2-(N-morpho-lino)ethane sulfonic acid) buffer.

To further illustrate, pharmaceutical acceptable carriers may include, for example, aqueous vehicles such as sodium chloride injection, Ringer's injection, isotonic dextrose injection, sterile water injection, or dextrose and lactated Ringer's injection, nonaqueous vehicles such as fixed oils of vegetable origin, cottonseed oil, corn oil, sesame oil, or peanut oil, antimicrobial agents at bacteriostatic or fungistatic concentrations, isotonic agents such as sodium chloride or dextrose, buffers such as phosphate or citrate buffers or MES (2-(N-morpholino)ethane sulfonic acid) buffers, antioxidants such as sodium bisulfate, local anesthetics such as procaine hydrochloride, suspending and dispersing agents such as sodium carboxymethylcelluose, hydroxypropyl methylcellulose, or polyvinylpyrrolidone, emulsifying agents such as Polysorbate 80 (TWEEN-80), sequestering or chelating agents such as EDTA (ethylenediaminetetraacetic acid) or EGTA (ethylene glycol tetraacetic acid), ethyl alcohol, polyethylene glycol, propylene glycol, sodium hydroxide, hydrochloric acid, citric acid, or lactic acid. Antimicrobial agents utilized as carriers may be added to pharmaceutical compositions in multiple-dose containers that include phenols or cresols, mercurials, benzyl alcohol, chlorobutanol, methyl and propyl p-hydroxybenzoic acid esters, thimerosal, benzalkonium chloride and benzethonium chloride. Suitable excipients may include, for example, water, saline, dextrose, glycerol, or ethanol. Suitable non-toxic auxiliary substances may include, for example, wetting or emulsifying agents, pH buffering agents, stabilizers, solubility enhancers, or agents such as sodium acetate, sorbitan monolaurate, triethanolamine oleate, or cyclodextrin.

The pharmaceutical compositions with the ACE2 polypeptide can be a liquid solution, suspension, emulsion, pill, capsule, tablet, sustained release formulation, or powder. Oral formulations can include standard carriers such as pharmaceutical grades of mannitol, lactose, starch, magnesium stearate, polyvinyl pyrollidone, sodium saccharine, cellulose, magnesium carbonate, etc.

The form of pharmaceutical compositions depends on a number of criteria, including, but not limited to, route of administration, extent of disease, or dose to be administered. The pharmaceutical compositions can be formulated for intravenous, oral, nasal, rectal, percutaneous, or intramuscular administration. For example, dosage forms for intravenous administration, may be formulated as lyophilized powder or fluid formulation; dosage forms for nasal administration may conveniently be formulated as aerosols, solutions, drops, gels or dry powders. In accordance to the desired route of administration, the pharmaceutical compositions can be formulated in the form of tablets, capsule, pill, dragee, powder, granule, sachets, cachets, lozenges, suspensions, emulsions, solutions, syrups, aerosols (as a solid or in a liquid medium), spray, inhalant, or suppository.

In some embodiments, the pharmaceutical compositions are formulated into an injectable composition. The injectable pharmaceutical compositions may be prepared in any conventional form, such as for example liquid solution, suspension, emulsion, or solid forms suitable for generating liquid solution, suspension, or emulsion. Preparations for injection may include sterile and/or non-pyretic solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use, and sterile and/or non-pyretic emulsions. The solutions may be either aqueous or nonaqueous. Aqueous is preferred.

In some embodiments, unit-dose i.v. or parenteral preparations are packaged in an ampoule, a vial, bag or a syringe with a needle. All preparations for parenteral administration should be sterile and not pyretic, as is known and practiced in the art.

Depending on the route of administration, the ACE2 polypeptide can be coated with or disposed in a selected material to protect it from natural conditions which may detrimentally affect its ability to perform its intended function. It may be administered alone, or in conjunction with a pharmaceutically acceptable carrier.

The invention further relates to a kit comprising containers with an ACE2 polypeptide.

The kit or container in a kit or pharmaceutical preparation preferably comprises the ACE2 polypeptide in an amount of 700 µg to 105 mg per container with ACE2 polypeptide.

The kit may comprise one or more than one container for each dose of ACE2 polypeptide.

A container for the pharmaceutical composition or in a kit may comprise one administration dose in a suitable encapsulation, such as a vial, flask, bag, syringe or the like.

The kit may comprise two or more such containers, wherein the containers are packaged together, e.g. in a packaging envelope such as a box or bag.

It is also possible to combine any compound of the invention with one or more additional active therapeutic agents in a unitary dosage form for simultaneous or sequential administration to a patient. The combination therapy may be administered as a simultaneous or sequential regimen.

Co-administration of the ACE2 polypeptide with one or more other active therapeutic agents is envisaged. Co-administration generally refers to simultaneous or sequential administration of the ACE2 polypeptide and one or more other active therapeutic agents, such that therapeutically effective amounts of the ACE2 polypeptide and one or more other active therapeutic agents are both present in the body of the patient.

Other therapeutic agents may be antiviral agents. E.g. antiviral agents that reduce SARS-CoV-2 proliferation in the subject. Other therapeutic agents may also be antibodies, e.g., antibodies to a coronavirus such as SARS-CoV2, or intravenous immunoglobulins (IVIG).

Co-administration includes administration of unit dosages of the compounds of the invention before or after administration of unit dosages of one or more other active therapeutic agents, for example, administration of the compounds of the invention within seconds, minutes, or hours of the administration of one or more other active therapeutic agents. For example, a unit dose of a compound of the invention can be administered first, followed within seconds or minutes by administration of a unit dose of one or more other active therapeutic agents. Alternatively, a unit dose of one or more other therapeutic agents can be administered first, followed by administration of a unit dose of a compound of the invention within seconds or minutes. In some cases, it may be desirable to administer a unit dose of a compound of the invention first, followed, after a period of hours (e.g., 1-12 hours), by administration of a unit dose of one or more other active therapeutic agents. In other cases, it may be desirable to administer a unit dose of one or more other active therapeutic agents first, followed, after a period of hours (e.g., 1-12 hours), by administration of a unit dose of a compound of the invention.

The invention also provides a nucleic acid encoding an ACE2 polypeptide according to the invention, e.g., DNA or RNA, in particular, an ACE2 polypeptide mutated to lack at least one of the glycosylation sites mentioned herein. Preferably, the nucleic acid is an expression vector, e.g., suitable for expression in a mammalian cell. The expression vector may also be suitable for expression in a bacterial cell, e.g., E.coli or in a yeast cell such as S. cerevisiae or S. pombe. Expression in a bacterial cell has the advantage that the protein is not glycosylated. A host cell comprising said nucleic acid is also provided.

Throughout the present disclosure, the articles "a", "an" and "the" are used herein to refer to one or to more than one (i.e., to at least one) of the grammatical object of the article.

As used herein, words of approximation such as, without limitation, "about", "substantial" or "substantially" refer to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by e.g. ±10%.

As used herein, the terms "include" and "including" have the same meaning as the terms "comprise" and "comprising". The terms "comprise" and "comprising" should be interpreted as being "open" transitional terms that permit the inclusion of additional components further to those components that are recited. "Comprising" in connection with a component connected to a range shall mean that further non-recited components are allowed but the recited component linked to that range shall be within said range and not outside said range. The terms "consist" and "consisting of" should be interpreted as being "closed" transitional terms that do not permit the inclusion of additional components other than the recited. The term "consisting essentially of" should be interpreted to be partially closed and allowing the inclusion only of additional components that do not fundamentally alter the nature of the recited subject matter, such as not permitting further non-recited active ingredients but allowing further non-recited auxiliary substances, like buffer components, fillers, and the like.

The present invention is further illustrated by the following examples, without being limited to these embodiments of the invention. All literature cited herein is herewith fully incorporated.

### Figures:

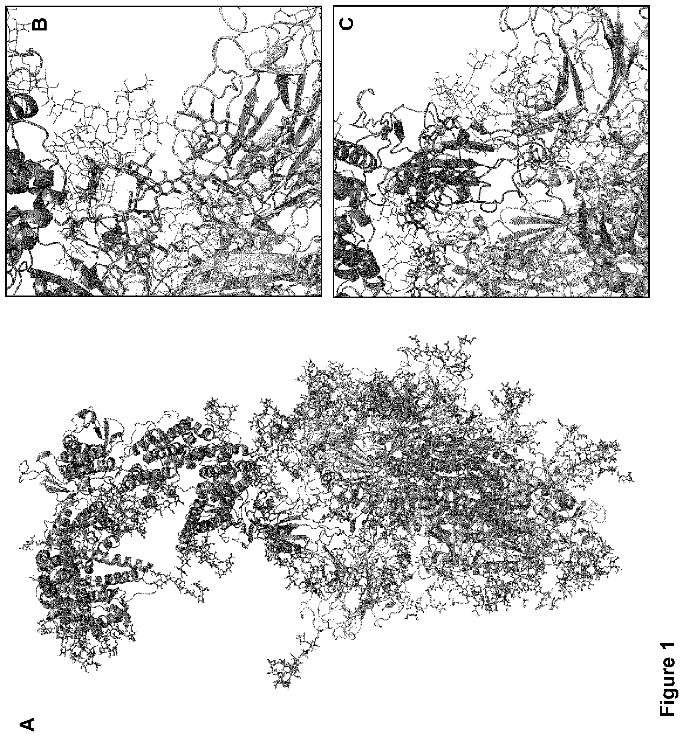

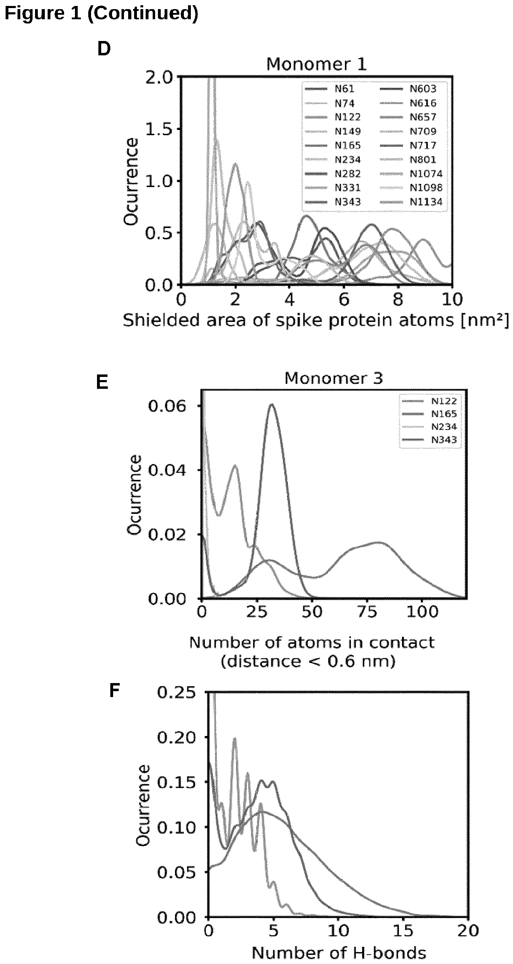

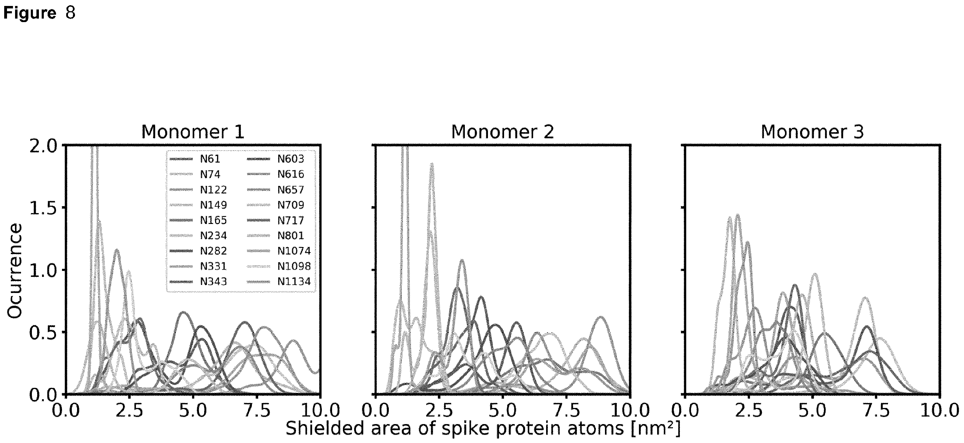

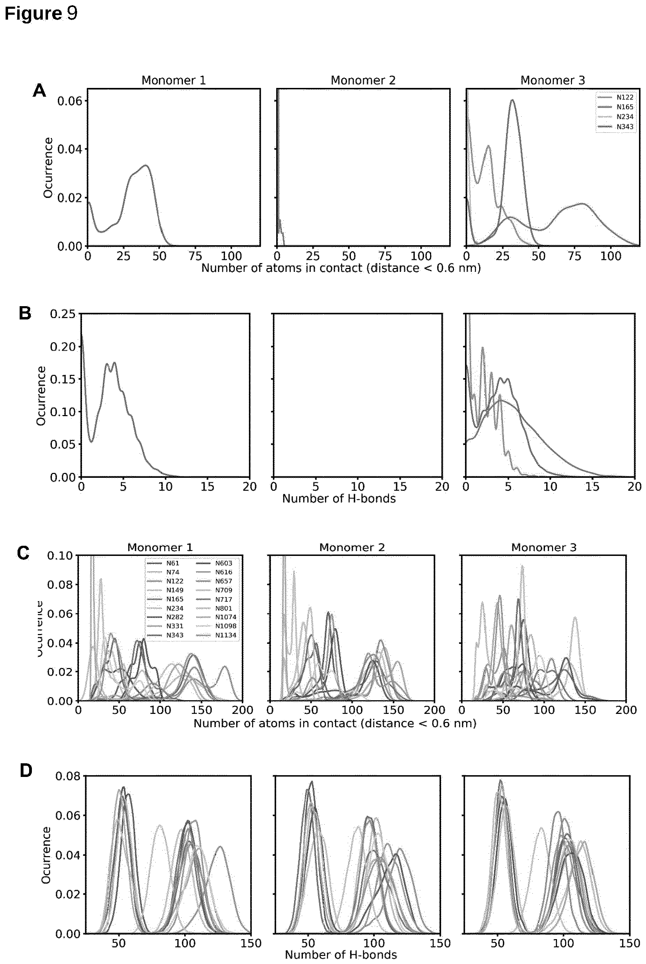

**Figure 1****. A 3D structural model of the glycosylated Spike-ACE2 complex. (A)** 3D model of the Spike trimer (in green, with RBD in dark green) binding to ACE2 (in grey) with complex glycosylation in magenta, Man5 glycans in light blue and Man9 glycans in orange. **(B)** Close-up view of the glycans at N122 (orange sticks) and N165 (dark green sticks) on monomer 3 of Spike. **(C)** Close-up view of the glycans at N331 (yellow) and N343 (purple) on monomer 3 of Spike. **(D)** Normalized distribution of the area of Spike protein atoms that is shielded by each of its glycans on monomer 1 (see fig. 8 for monomers 2 and 3). **(E)** Normalized distribution of the number of atoms in contact with ACE2 and **(F)** the number of hydrogen bonds with ACE2 for glycans on monomer 3 of Spike (see fig. 9 for monomers 1 and 2).

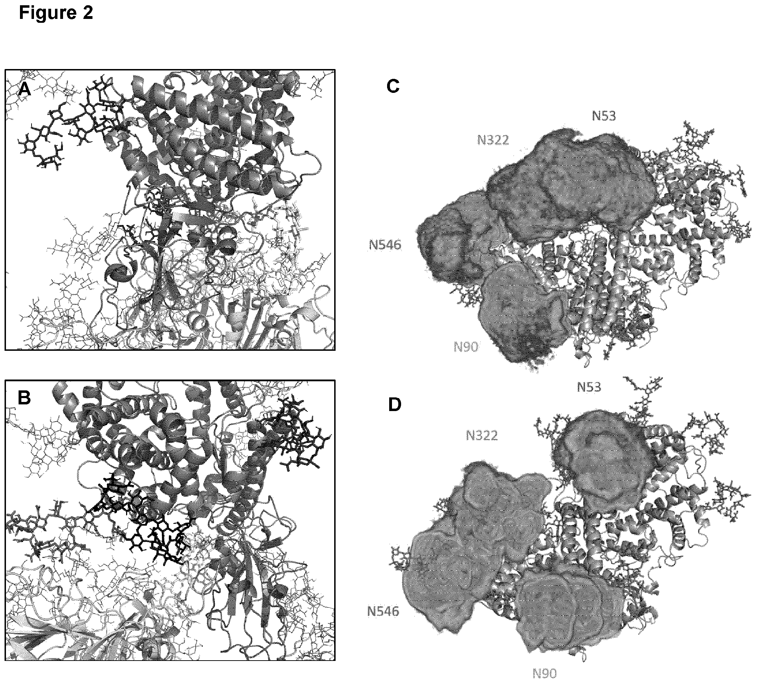

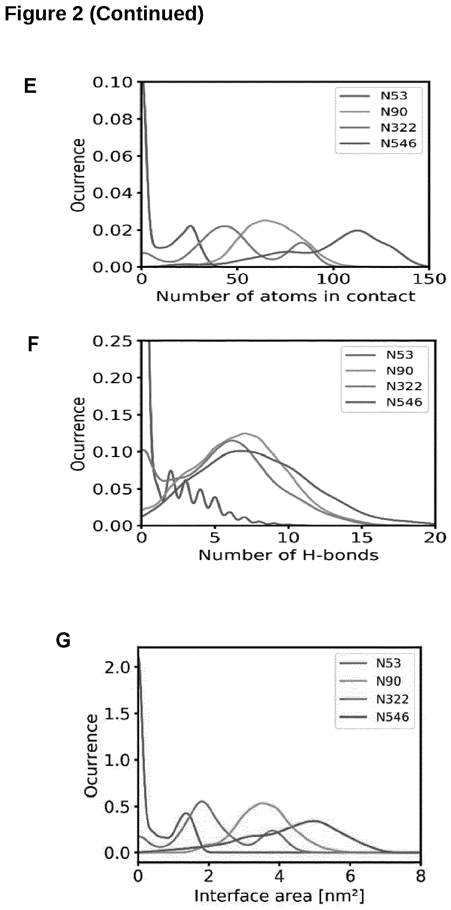

**Figure 2****. Critical glycans at the Spike-ACE2 binding interface. (A)** and **(B)** Glycans at the Spike-ACE2 interface. Spike protein atoms are shown in green with the RBD in dark green. ACE protein atoms are indicated in grey. Complex glycosylation is shown in magenta, Man5 N-glycans in light blue and Man9 N-glycans in orange. Glycans of ACE2 at N53, N90, N322 and N546 are shown in blue, yellow, black and red, respectively. **(C)** and **(D)** Average location heat map of glycans at N53, N90, N322 and N546 from **(C)** free ACE2 and **(D)** ACE2 in complex with Spike. Blue color indicates regions in space that are less frequently occupied by glycan atoms, red color indicates regions in space that are more frequently occupied by glycan atoms. **(E)** Normalized distribution of the number of atoms of glycans at N53, N90, N322 and N546 of ACE2 that are in contact with Spike (distance < 0.6 nm). **(F)** Normalized distribution of the number of hydrogen bonds between glycans at N53, N90, N322, N546 of ACE2 and Spike. **(G)** Normalized distribution of the interface area between Spike and glycans at N53, N90, N322 and N546 of ACE2.

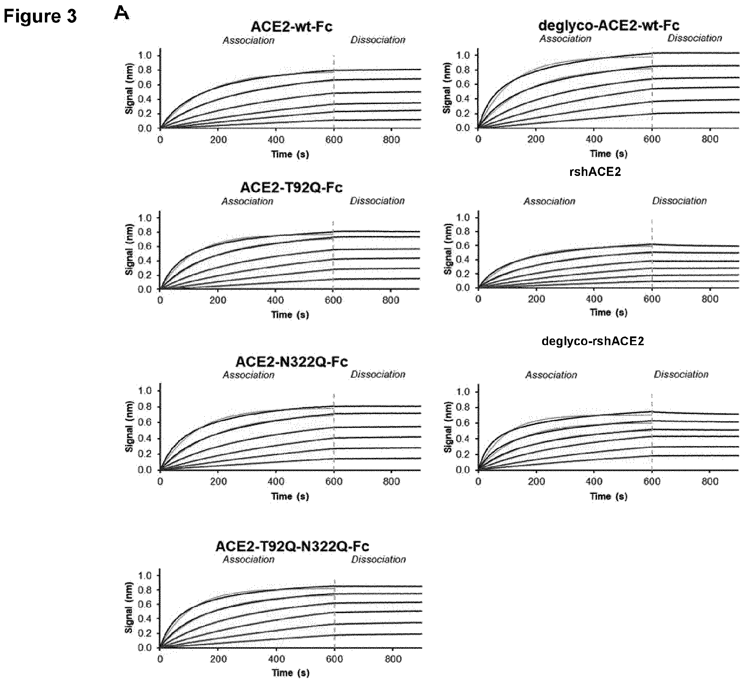

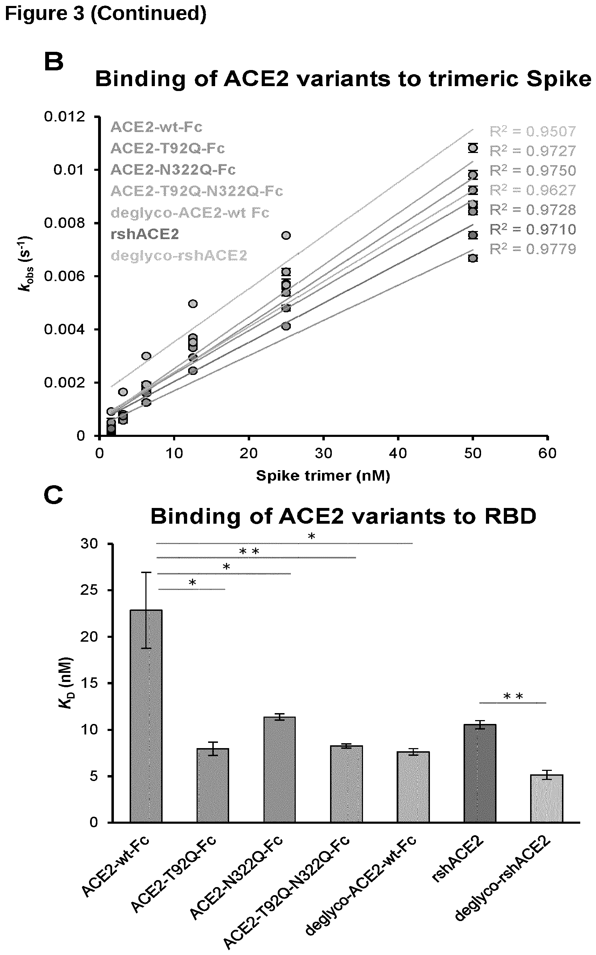

**Figure 3****. Binding of Spike and RBD to glyco-engineered ACE2 variants. (A,B)** Binding of Spike to glyco-engineered ACE2 variants as determined by biolayer interferometry (BLI). **(A)** Binding of ACE2 variants to immobilized trimeric Spike analyzed by BLI. Black lines represent the response curves of the association and dissociation phases. Red lines represent the 1:1 binding model for association used to determine *k*_{obs} values. For each ACE2 variant one representative experiment out of three is shown. **(B)** Plots of *k*_{obs} (observed association rate) as a function of Spike concentration were generated by fitting the association data to a 1:1 binding model. Binding analysis was performed by dipping ACE2 loaded biosensors into 2-fold serial dilutions of purified Spike (50 nM-1.6 nM). **(C)** *K*_{D} values for the interaction of the indicated glyco-engineered ACE2 variants with monomeric RBD. Binding of clinical grade rshACE2 and deglycosylated rshACE2 to Spike and RBD is also shown. Data are shown as mean +/- SEM of 3-5 independent experiments. *P<0.05; **P<0.01 (Student's t-test).

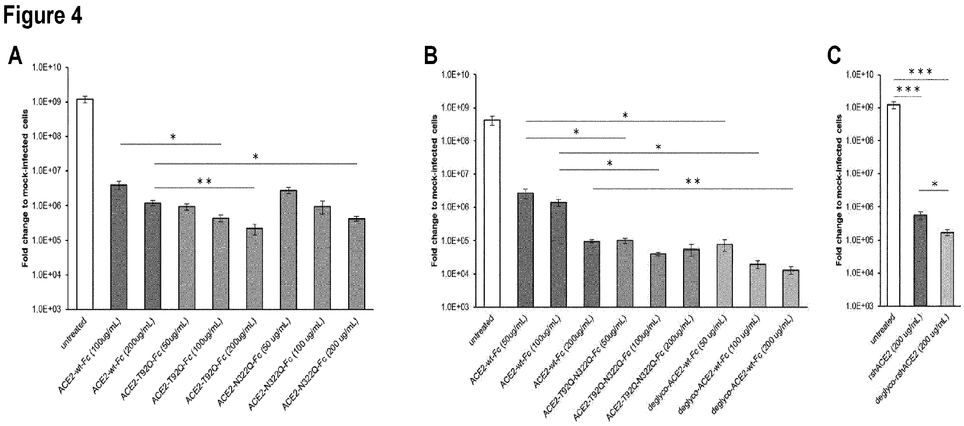

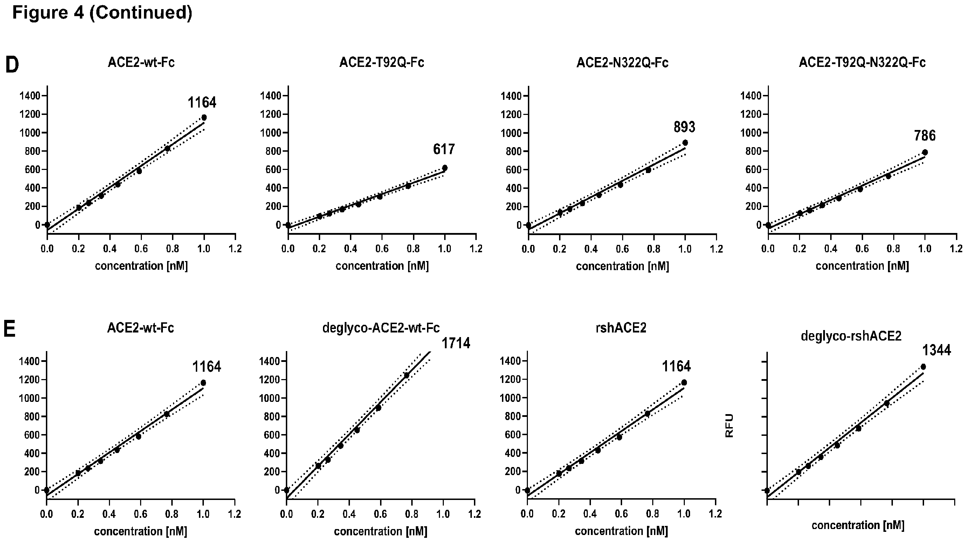

**Figure 4****. Critical role of ACE2 glycosylation in SARS-CoV-2 infectivity. (A) and (B)** Inhibition of SARS-CoV-2 infection of Vero E6 cells using wild type ACE2-Fc, the indicated glyco-engineered ACE2-Fc mutants, and deglycosylated ACE2-Fc, used at the indicated concentrations. **(C)** Inhibition of SARS-CoV-2 infection of Vero E6 cells using clinical grade rshACE2 and deglycosylated rshACE2. In (A-C) viral RNA levels were determined by RT-qPCR 15 hours after inoculation with SARS-CoV-2 (Swedish isolate, 10⁶ PFU; MOI 20). Data are presented as fold changes of viral loads over mock-treated (untreated) controls (mean log10 values ± SD). *P<0.05; **P<0.01; ***P<0.001 (Student's t-test). **(D) and (E)** Enzymatic activity of ACE2-Fc variants, deglycosylated (deglyco) ACE2-wt-Fc, rshACE2 and deglyco-rshACE2 was quantified using 100 µM 7-methoxycoumarin-4-yl-acetyl-Ala-Pro-Lys-2,4-dinitrophenyl as substrate. Proteolytic activity is plotted as relative fluorescence units (RFU) over ACE2 concentration (in nM). One representative experiment out of two is shown.

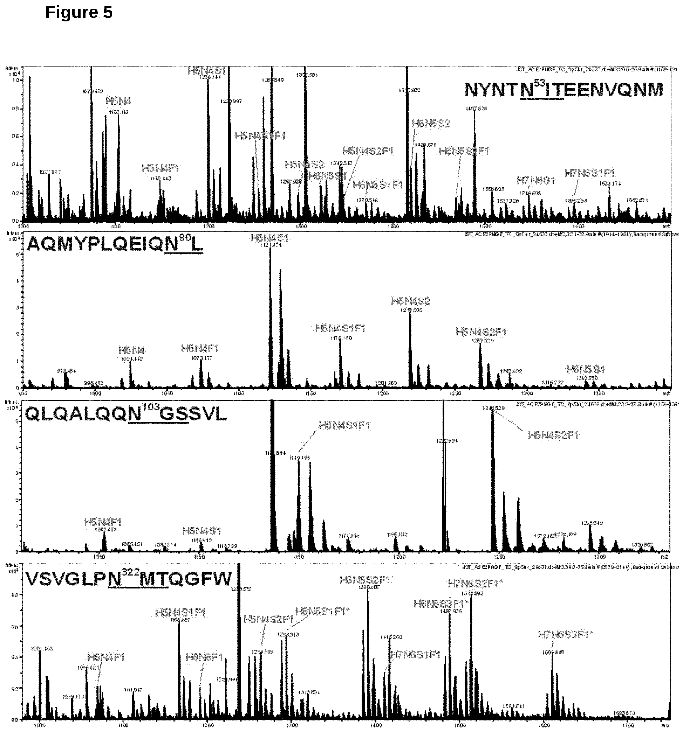

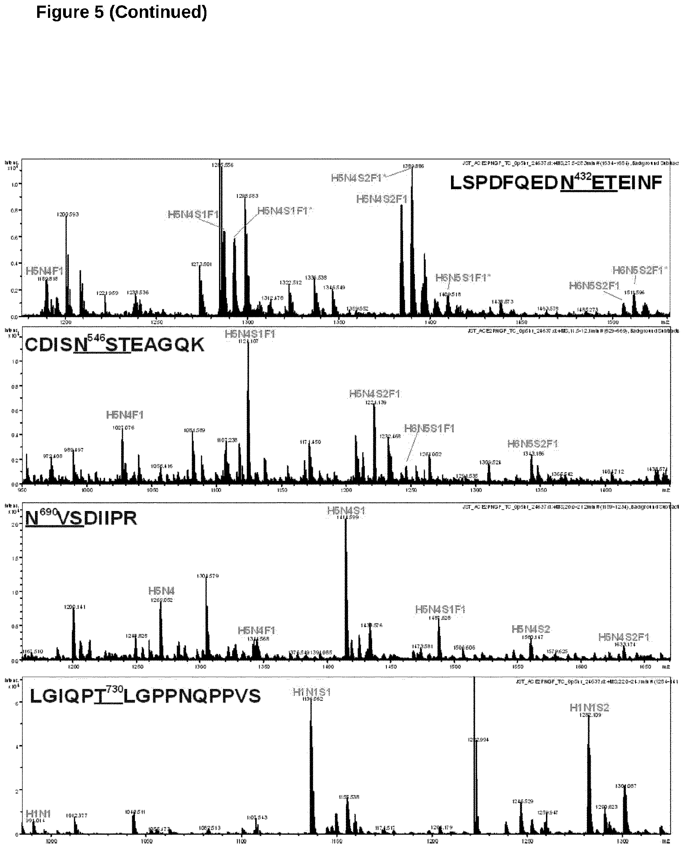

**Figure 5****. Site-specific glycosylation profiles of rshACE2.** Prior to analysis by LC-ESI-MS, reduced and S-alkylated rshACE2 was digested in-solution with chymotrypsin and trypsin. Glycan compositions are denoted as the sum of hexoses (H), N-acetyl-hexosamines (N), N-acetyl-neuraminic acids (i.e. sialic acids; S) and fucoses (F). Molecular ion-species detected as ammonium adducts (i.e. +17 amu) are indicated by asterisks.

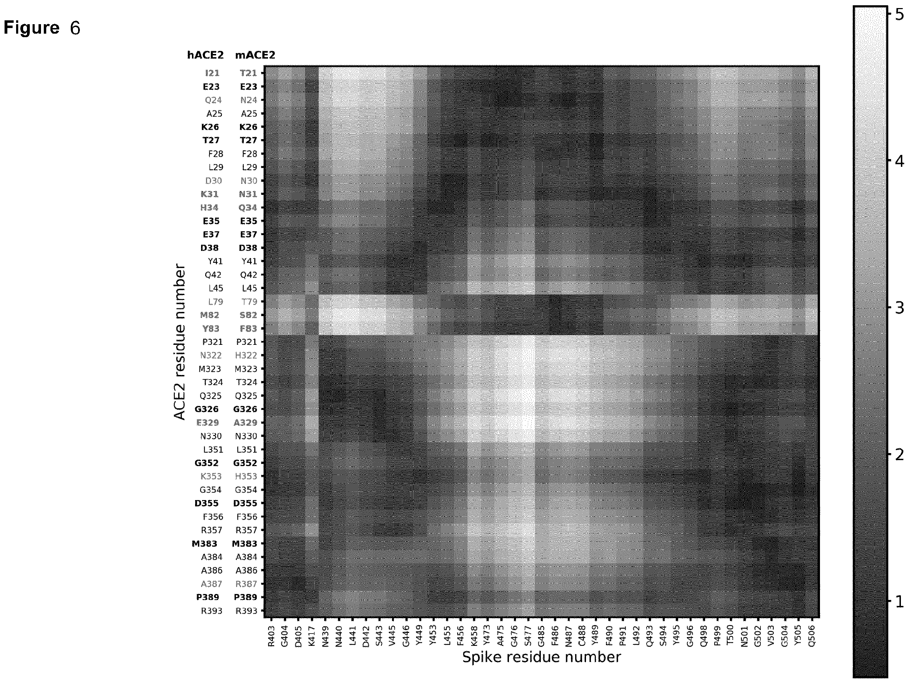

**Figure 6****. Distance heatmap of human ACE2 (hACE2) residues in contact with Spike RBD.** Black squares correspond to an average distance of 0 nm and white squares to an average distance of 6 nm. Residues which differ in mouse ACE2 (mACE2) are highlighted in red, residues known to be genetically polymorphic (52) are shown in bold.

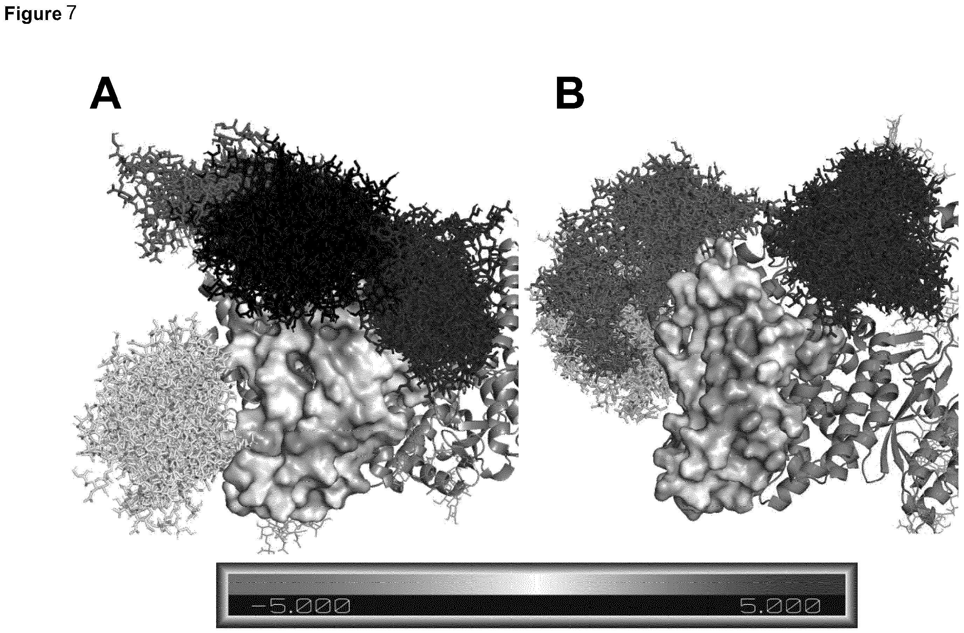

**Figure 7****. Electrostatic potential of the binding interface region on ACE2.** Electrostatic potential surface of **(A)** human ACE2 and **(B)** mouse ACE2. Red corresponds to a negative charge and blue to a positive charge. Note the more distinct pattern of negative and positive charges in the mouse ACE2 protein, leading to a mismatch for interactions with the mostly neutral residues at the interface of Spike (see fig. 6). A bundle of glycan conformations is shown in sticks for the glycans at N53 (blue), N90 (yellow), N322 (black) and N546 (red) in human ACE2 and at N53 (blue), N536 (green), and N546 (red) in mouse ACE2.
**Figure 8****. Normalized distribution of the area of Spike atoms shielded by each of its glycans.** The distributions were obtained from the molecular dynamics simulations for the individual monomers of Spike. For every Spike glycosite, the area of Spike protein atoms that is shielded by the glycan during the simulation is indicated. For instance, the glycan at N331 on monomer 1 shields between 7.5 and 10.0 nm² of the Spike surface (see also Fig. 1C).
**Figure 9****. Interactions of glycans of Spike with ACE2 and with Spike. (A)** Normalized distribution of the number of atoms of glycans of the indicated Spike monomers that are in contact with human ACE2. **(B)** Normalized distribution of the number of hydrogen bonds formed between Spike glycans and human ACE2, shown for 2 Spike monomers. Glycans of the second monomer did not form H-bonds with human ACE2. **(C)** Normalized distribution of the number of atoms of Spike glycans that are in contact with Spike protein atoms, shown for all 3 Spike monomers. **(D)** Normalized distribution of the number of hydrogen bonds formed between Spike glycans and Spike for all 3 Spike monomers. Monomer 3 is the interacting monomer containing the RBD. The number of interacting atoms and the number of hydrogen bonds observed during the MD simulations are represented as normalized distribution for the individual glycosites on Spike on the different monomers.

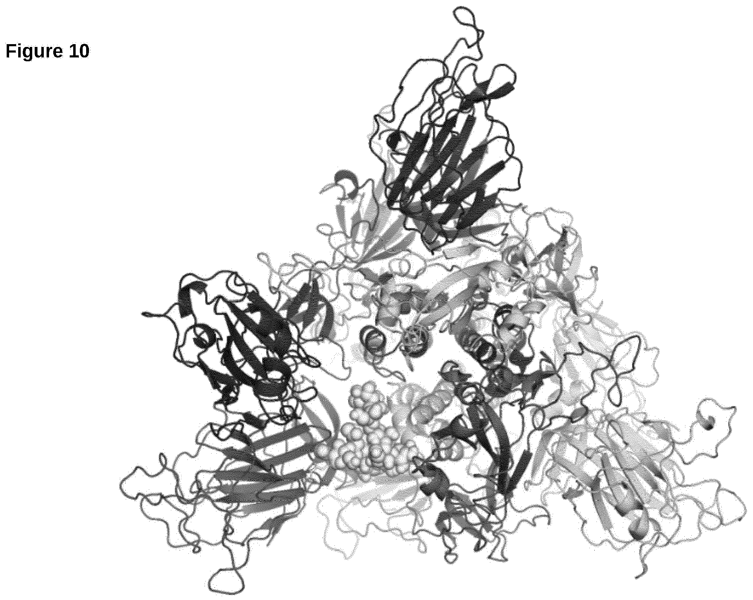

**Figure 10****.** **3D** **positional modelling of glycan N234 in the Spike trimer.** Glycan at N234 (yellow spheres) of monomer 2 (red) is partially inserting into the core of the Spike trimer. The vacant space in the core is created by the receptor-binding domain of monomer 3 (blue) residing in the up conformation. Monomer 1 is shown in green.

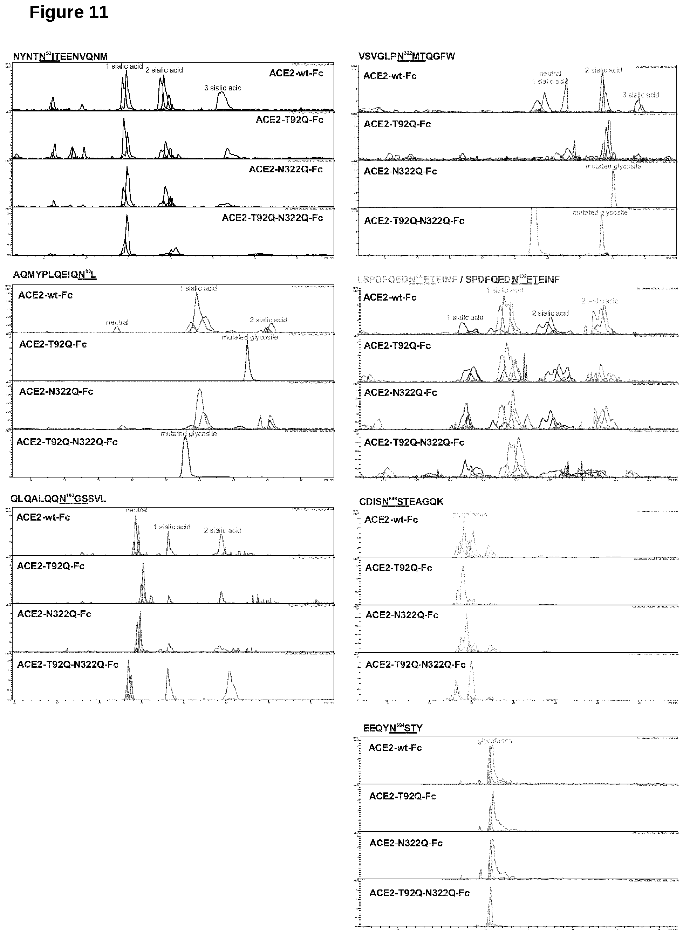

**Figure 11****. Site-specific ablation of ACE2 glycans by mutagenesis.** Prior to analysis by LC-ESI-MS(/MS), the indicated reduced and S-alkylated ACE2-Fc variants were digested in-solution with chymotrypsin and trypsin. Comparative analysis of extracted ion chromatograms of glycopeptides confirmed the absence of glycans attached to N90 and/or N322 in ACE2-T92Q-Fc, ACE2-N322Q-Fc and ACE2-T92Q-N322Q-Fc. The peptide sequences surrounding the different glycosites are shown.

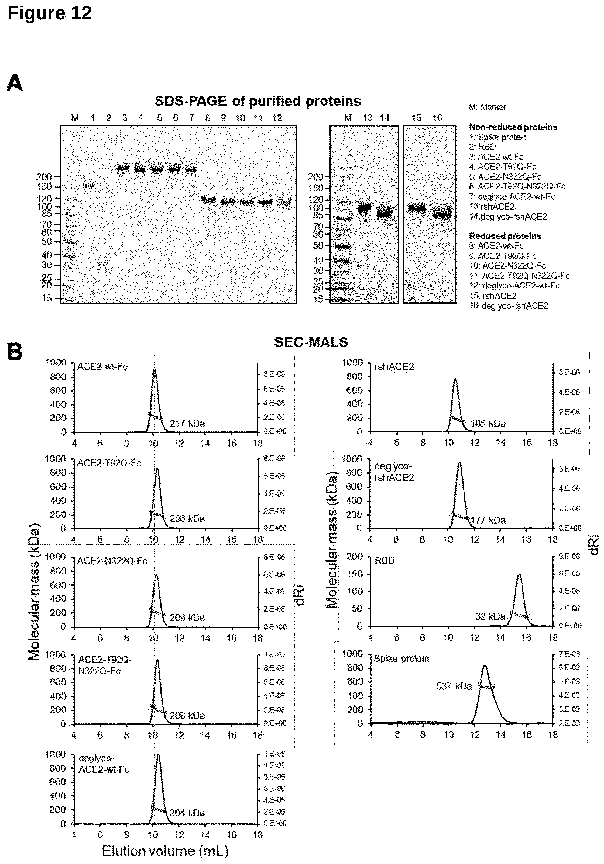

**Figure 12****. Analysis of recombinantly expressed and purified proteins. (A)** Analysis of 1 µg of each purified protein by SDS-PAGE. M, molecular mass markers. **(B)** SEC-MALS analysis of purified proteins. A total of 50 µg of Spike was loaded onto a Superose 6 Increase 10/300 GL column (Cytiva, United States) at a flow rate of 0.25 mL min⁻¹. All other proteins were analyzed by injection of a total of 25 µg of the respective protein onto a Superdex 200 10/300 GL column (Cytiva, United States) at a flow rate of 0.75 mL min⁻¹. The elution time of ACE2-wt-Fc is marked with a dashed line. Molecular masses (red traces) were determined by MALS and calculated using the ASTRA software. dRI, change in refractive index.

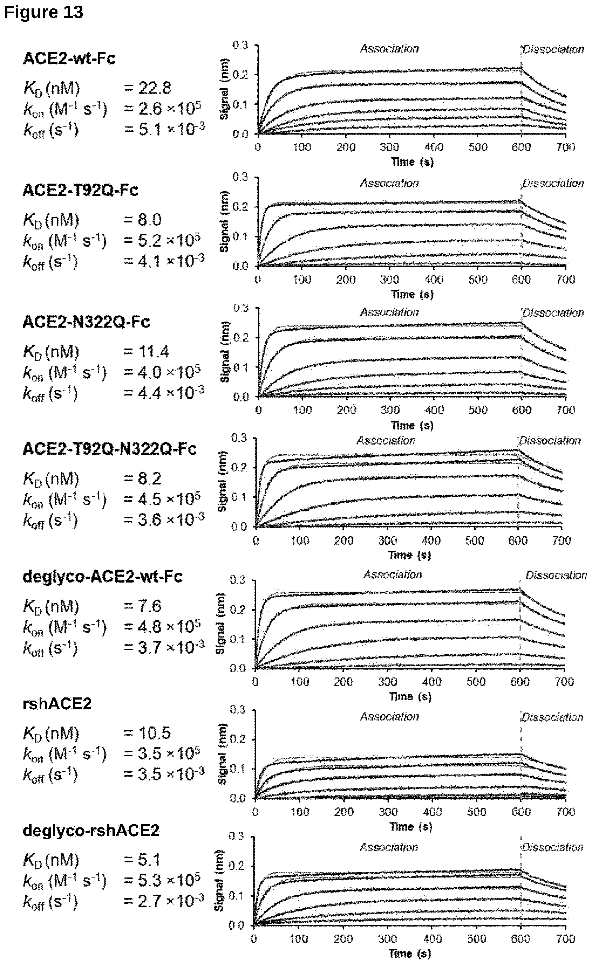

**Figure 13****. Binding kinetics of the interaction between human ACE2 variants and RBD obtained by biolayer interferometry (BLI) analysis.** Biosensors loaded with ACE2-wt-Fc were submerged into 2-fold (200 nM-6.25 nM) serial dilutions of RBD. Biosensors loaded with all other ACE2 variants were dipped into 3-fold (200 nM-0.8 nM) serial dilutions of RBD. Black lines represent the response curves of the association and dissociation phases. The data were fitted to a 1:1 binding model (red lines). For each ACE2 variant one representative experiment is shown. K_{D}, kₒₙ and k_{off} values were calculated by the Octet data analysis software and represent the mean of 3-5 independent experiments.

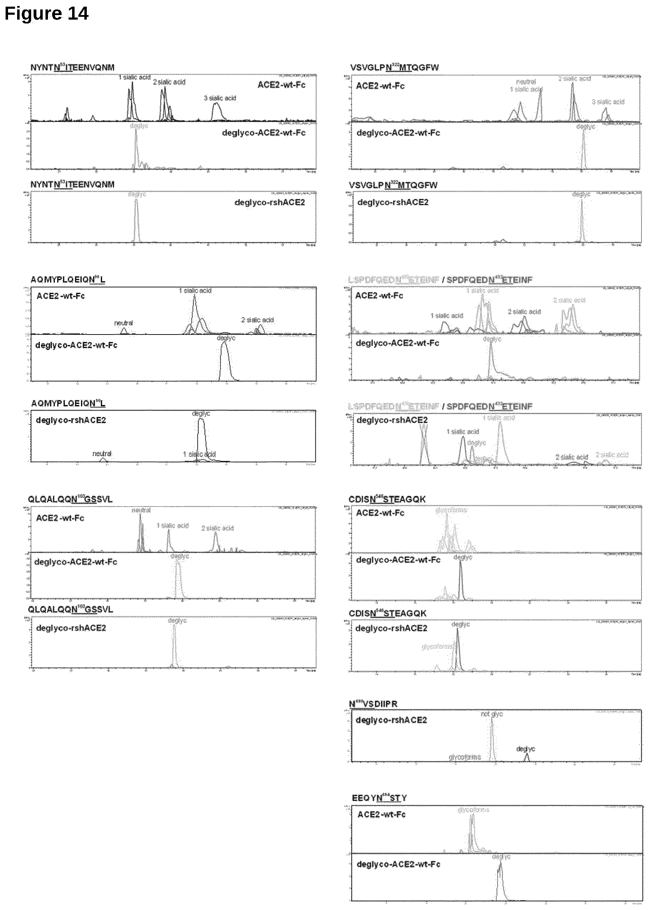

**Figure 14****. Deglycosylation of ACE2-wt-Fc and rshACE2 by PNGase F.** Prior to analysis by LC-ESI-MS(/MS), native and deglycosylated (deglyco) ACE2-wt-Fc and rshACE2 were reduced, alkylated and then digested in-solution with chymotrypsin and trypsin. Comparative analysis of extracted ion chromatograms of glycopeptides confirmed the absence of N-glycans attached to N53, N90, N103 and N322 in deglyco-ACE2-wt-Fc and deglyco-rshACE2. Peptide sequences surrounding the different glycosites are shown.

### Examples:

### Example 1: Modeling of the Spike-hACE2 complex

To model the fully glycosylated SARS-CoV-2 Spike-human ACE2 (hACE2) complex, a protein model was created using partial experimental structures deposited in the protein databank (PDB). The Spike RBD domain in complex with hACE2 (PDB: 6M17) was superimposed with the opened RBD domain in a Spike structure with one open RBD domain (PDB: 6VYB). Missing residues in Spike were modeled using SWISS-MODEL (Waterhouse et al. Nucleic Acids Res 46, W296-W303 (2018)) and the superimposed structure as template based on the complete SARS-CoV-2 S sequence (GenBank QHD43416.1).

Different types of glycans were added to Spike and hACE2. For Spike, the assignments of Watanabe et al., Science 369, 330-333 (2020), were followed, selecting oligomannosidic (Man5 or Man9) or complex (bi-antennary di-sialylated core fucosylated; NaNaF) glycans according to the majority of the glycans detected at the respective site. For hACE2, complex (i.e. bi-antennary di-sialylated core fucosylated) N-glycans were added. See Table 2 for the exact assignments. Initial conformations of the glycans were selected following the formulas outlined in Turupcu et al., J Chem Inf Model 57, 2222-2236 (2017). In brief, molecular dynamics simulations were performed of mini-peptides with the glycans attached. Local Elevation (Huber et al., J. Comp-Aided Mol. Des. 8, 695-708 (1994)) was used to enhance the sampling of all glycosidic linkages, during simulations of 100 ns. The entire glycan trees were clustered based on the conformations of the individual glycosidic linkages. This resulted in conformational bundles containing 1301, 1340 and 2413 distinct conformations of Man5, Man9 and NaNaF, respectively. These conformations were fitted onto the respective glycosylation site in the Spike-hACE2 complex using a superposition of the backbone of the asparagine residues and the non-bonded interaction energy between the glycan and protein atoms or previously added glycans was computed. The lowest energy conformation was retained. Topologies and initial conformations were generated using the gromos++ suite of pre- and post-MD tools (Eichenberger et al., J Chem Theory Comput 7, 3379-3390 (2011)). Glycans were added to the complex sequentially, to avoid collisions between individual glycans. A few modelled glycans were incompatible with loops of the Spike protein not resolved in the experimental structures. Loops involved in these structural incompatibilities (residues 141-165 and 471-490) were partially remodelled in the fully glycosylated model using the RCD+ loop modelling server (Lopez-Blanco et al., Nucleic Acids Res 44, W395-400 (2016)). The final model was energy-minimized with the GROMOS 54A8 protein force-field (Reif et al., J Chem Theory Comput 8, 3705-3723 (2012)), the GROMOS 53A6glyc glycan force-field (Pol-Fachin et al., J Comput Chem 35, 2087-2095 (2014)) and the GROMOS simulation software using the steepest decent algorithm (Schmid et al., Comput Phys Commun 183, 890-903 (2012)).

**Table 2: Glycan structures used for modelling of SARS-CoV-2 Spike and human ACE2. Glycosylation sites and glycan structures are indicated. Blue squares indicate N-acetylglucosamine (GlcNAc), green circles mannose, yellow circles galactose, purple squares sialic acid and red triangles fucose residues. Anomericity and positions of glycosidic linkages are indicated where necessary. The GlcNAc to the right is connected to the Asn (N) residue of the protein at the indicated glycosylation site.**

| **Protein** | **Glycosylation site** | **Glycan structure** |
|---|---|---|
| **SARS-CoV-2 S** | N61 | 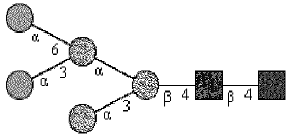 |
| | N74 | 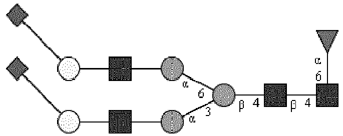 |
| | N122 | 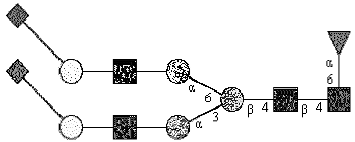 |
| | N149 | 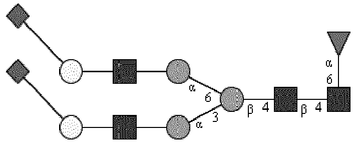 |
| | N165 | 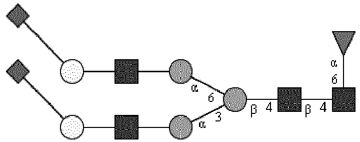 |
| | N234 | 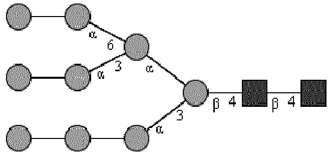 |
| | N282 | 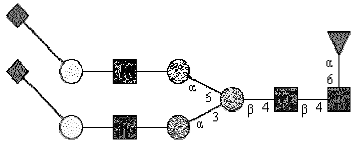 |
| | N331 | 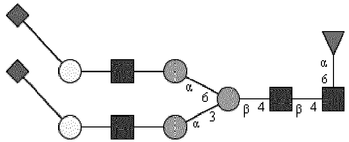 |
| | N343 | 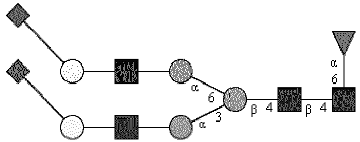 |
| | N603 | 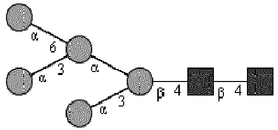 |
| | N616 | 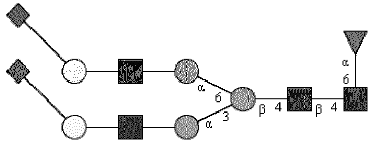 |
| | N657 | 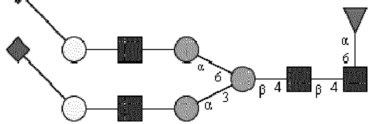 |
| | N709 | 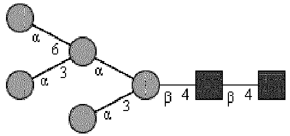 |
| | N717 | 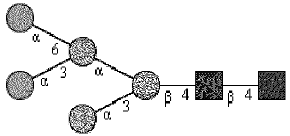 |
| | N801 | 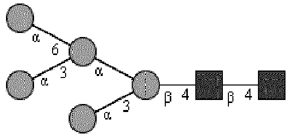 |
| | N1098 | 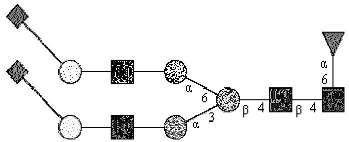 |
| | N1134 | 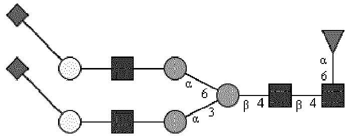 |
| **hACE2** | N53 | 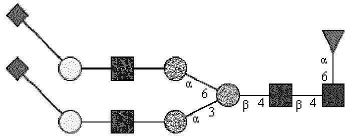 |
| | N90 | 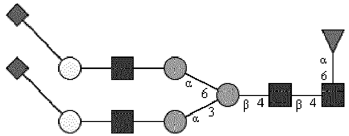 |
| | N103 | 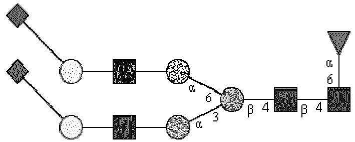 |
| | N322 | 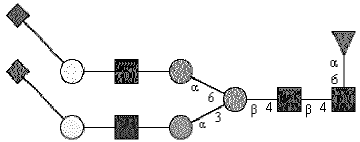 |
| | N432 | 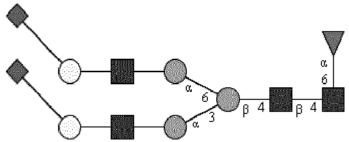 |
| | N546 | 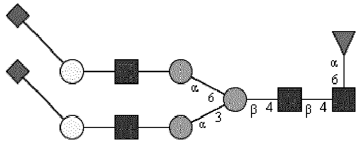 |
| | N690 | 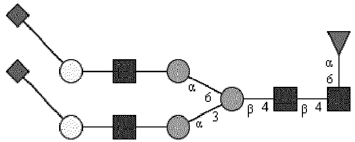 |

### Example 2: Molecular dynamics simulations

Molecular dynamics simulations were performed using the simulation package Gromacs (Version 2019.5) and the indicated force field parameters. hACE2 was reduced to residues 21 to 730 in the models, to reduce its overall size prior to simulation. The models were placed in rhombic dodecahedron simulation boxes and solvated by explicit SPC water molecules (Berendsen et al., Intermolecular Forces, 331-342 (1981)). This resulted in simulation systems of 5.9 × 10⁵ and 2.2 × 10⁶ atoms for hACE2 and the Spike-hACE2 complex, respectively. Five independent 50-ns molecular dynamics simulations were performed for ACE2 and two independent 50-ns simulations for the Spike-hACE2 complex. The equations of motion were integrated using a leapfrog integration scheme (Hockney et al., Methods in Computational Physics 9, 135-211 (1970)) with a time-step of 2 fs. Non-bonded interactions were calculated within a cutoff sphere of 1.4 nm and electrostatic interactions were computed using a particle-particle particle-mesh (P3M) approach (Hockney & Eastwood, Computer simulations using particles. CRC Press (1988)). Bond-lengths were constrained to their optimal values using the Lincs algorithm (Hess et al, J Comput Chem 18, 1463-1472 (1997)). Temperature was maintained at a constant value using a velocity-rescaling algorithm (Berendsen et al., J Chem Phys 81, 3684-3690 (1984)) with a relaxation time of 0.1 ps. Pressure was maintained constant using a Parrinello-Rahman barostat (Parrinello & Rahman, J Applied Physics 52, 7182-7190 (1981)) with a relaxation time of 2.0 ps and an estimated isothermal compressibility of 4.5 × 10⁻⁵ bar⁻¹. Configurations were stored every 10 ps for subsequent analyses. Hydrogen bonds were identified using a geometric criterion. A hydrogen bond was logged if the donor-acceptor distance is within 0.25 nm and the donor-hydrogen-acceptor angle was larger than 135 degrees. The solvent accessible surface area was determined by rolling a probe with diameter 0.14 nm over the surface of the protein, using slices of 0.005 nm width. An atom contact was assigned if the distance between two atoms were within 0.6 nm. The distributions of atom contacts, hydrogen bonds and solvent accessible surface area were estimated using a kernel density estimator with gaussian kernels. Glycan densities were calculated using the program iondens of the gromos++ suite.

### Example 3: Recombinant expression of proteins

Soluble recombinant human ACE2 (rshACE2) was provided by Apeiron Biologicals (Vienna, Austria). Recombinant expression of all other proteins was performed by transient transfection of HEK293-6E cells, licensed from National Research Council (NRC) of Canada, as previously described (Durocher et al., Nucleic Acids Res 30, E9 (2002)). Cells were cultivated in FreeStyle F17 expression medium supplemented with 0.1% (v/v) Pluronic F-68 and 4 mM L-glutamine (all from Thermo Fisher Scientific, United States) in shaking flasks at 37°C, 8% CO₂, 80% humidity and 130 rpm in a Climo-Shaker ISF1-XC (Adolf Kühner AG, Switzerland). pCAGGS vector constructs containing either the sequence of the SARS-CoV-2 RBD (residues R319-F541) or the complete luminal domain of Spike, modified in terms of removal of the polybasic furin cleavage site and introduction of two stabilizing point mutations (K986P and V987P), were kindly provided by Florian Krammer, Icahn School of Medicine at Mount Sinai (New York, United States) (Amanat et al., Nat Med 26, 1033-1036 (2020)). Plasmid constructs pcDNA3-sACE2(WT)-Fc(IgG1) and pcDNA3-sACE2-T92Q-Fc(IgG1) were obtained from Addgene (United States). The N322Q mutation was introduced into ACE2-wt-Fc and ACE2-T92Q-Fc using the QuikChange Lightning Site-Directed-Mutagenesis kit (Agilent Technologies, United States) according to the manufacturer's instructions and the respective parental vector as template. High quality plasmid preparations for expression of ACE2-Fc variants were prepared using the PureYield Plasmid Midiprep System (Promega, United States). Transient transfection of the cells was performed at a cell density of approximately 1.7×10⁶ cells mL⁻¹ culture volume using a total of 1 µg of plasmid DNA and 2 µg of linear 40-kDa polyethylenimine (Polysciences Inc., Germany) per mL culture volume. 48 h and 96 h after transfection, cells were supplemented with 0.5% (w/v) tryptone N1 (Organotechnie, France) and 0.25% (w/v) D(+)-glucose (Carl Roth, Germany). Soluble proteins were harvested after 120-144 h by centrifugation (10 000 g, 15 min, 4°C).

### Example 4: Purification of recombinantly expressed proteins

After filtration through 0.45 µm membrane filters (Merck Millipore, Germany), supernatants containing RBD or soluble Spike were concentrated and diafiltrated against 20 mM sodium phosphate buffer containing 500 mM NaCl and 20 mM imidazole (pH 7.4) using a Labscale TFF system equipped with a 5 kDa cut-off PelliconTM XL device (Merck, Germany). The His-tagged proteins were captured using a 5 mL HisTrap FF crude column connected to an ÄKTA pure chromatography system (both from Cytiva, United States). Bound proteins were eluted by applying a linear gradient of 20 to 500 mM imidazole over 20 column volumes. ACE2-Fc variants were purified by affinity chromatography using a 5 mL HiTrap Protein A column (Cytiva, United States) according to the manufacturer's instructions and 0.1 M glycine-HCl (pH 3.5) for elution. Eluate fractions were immediately neutralized using 2 M Tris (pH 12.0). Fractions containing the protein of interest were pooled, concentrated using Vivaspin 20 Ultrafiltration Units (Sartorius, Germany) and dialyzed against PBS (pH 7.4) at 4°C overnight using SnakeSkin Dialysis Tubing (Thermo Fisher Scientific, United States). The RBD was further purified by size exclusion chromatography (SEC) using a HiLoad 16/600 Superdex 200 pg column (Cytiva, United States) eluted with PBS. All purified proteins were stored at -80°C until further use.

### Example 5: PNGase F digest of ACE2-wt-Fc and rsh-ACE2

For deglycosylation of ACE2-wt-Fc and rsh-ACE2, proteins (2 mg mL⁻¹) were incubated with 180000 U mL⁻¹ PNGase F (New England Biolabs, Unites States) in PBS (pH 7.4) for 24 h at 37°C. Deglycosylated proteins were purified by preparative SEC using a HiLoad 16/600 Superdex 200 pg column eluted in PBS. Deglycosylation of PNGase F digested ACE2 variants was assessed by LC-ESI-MS(/MS) (fig. 13), SDS-PAGE and SEC-MALS (fig. 14).

### Example 6: Bio-Layer Interferometry (BLI) measurements

Interaction studies were performed on an Octet RED96e system using high precision streptavidin (SAX) biosensors (both from ForteBio, United States). Thus, all capture molecules (ACE2-wt-Fc, ACE2-T92Q-Fc, ACE2-N322Q-Fc, ACE2-T92Q-N322Q-Fc, deglyco-ACE2-wt-Fc, rshACE2 and deglyco-rshACE2) were biotinylated using the EZ-Link Sulfo-NHS-LC-Biotin kit (Thermo Fisher Scientific, United States). Excess sulfo-NHS-LC-biotin was quenched by adding Tris-HCl buffer (800 mM, pH 7.4) to a final concentration of 3 mM. Biotinylated proteins were further purified using PD-10 desalting columns (Cytiva, United States) according to the manufacturer's protocol. All assays were conducted in PBS supplemented with 0.05% (v/v) Tween 20 and 1% (w/v) BSA (PBST-BSA) at 25°C with the plate shaking at 1000 rpm. The SAX biosensors were first equilibrated in PBST-BSA and then dipped into a 34 nM solution of the respective biotinylated capture molecule until a signal threshold of 0.8 nm was reached. Subsequently, the biosensors were dipped into PBST-BSA for 90 sec to record a baseline, before they were submerged into different concentrations of RBD or the Spike protein to record association rates. For binding analysis of trimeric Spike, all biosensors were dipped into 2-fold serial dilutions of the protein (50 nM-1.6 nM). To determine KD values, titration of RBD was performed at different concentrations to cover a broad concentration range around the respective KD value (48). Biosensors loaded with ACE2 variants were submerged into 2-fold (200 nM-6.25 nM) or 3-fold (200 nM-0.8 nM) serial dilutions of RBD as appropriate for 600 sec. For dissociation, the biosensors were dipped into PBST-BSA for 300 sec (for analysis of Spike) or 100 sec (for analysis of RBD). Each experiment included a baseline measurement using PBST-BSA (negative control) as well as a positive control (RBD). Of note, no unspecific binding of RBD or the Spike protein to SAX biosensors was observed. Data were evaluated under consideration of the limit of detection (LOD) and limit of quantification (LOQ) as reported elsewhere (Armbruster et al., Clin Biochem Rev 29 Suppl 1, S49-52 (2008)). Each experiment was performed 3-5 times. Analysis was performed using the Octet data analysis software version 11.1.1.39 (ForteBio, United States) according to the manufacturer's guidelines.

### Example 7: SDS-PAGE

SDS-PAGE was carried out using a 4-15% MINI-PROTEAN TGX Stain-Free Protein Gel, the Mini-PROTEAN Tetra Vertical Electrophoresis Cell (both from Bio-Rad Laboratories Inc., United States) and SDS-PAGE running buffer (20 mM Tris, 200 mM glycine, 0.1% (w/v) SDS). 1 µg of each purified protein was mixed with SDS sample buffer (62.5 mM Tris/HCl (pH 6.8), 2.5% (w/v) SDS, 10% (w/v) glycerol, 0.01% (w/v) bromophenol blue), heated to 70°C for 10 min and loaded onto the gel. For reducing conditions purified samples were mixed with SDS-PAGE sample buffer containing 0.75 M β-mercaptoethanol and heated to 95°C for 5 min prior to loading. The PageRuler Unstained Protein Ladder (Thermo Fisher Scientific, United States) was used as a size marker. Protein bands were visualized with the Gel Doc XR+ Imager (Bio-Rad Laboratories Inc., United States).

### Example 8: Size-Exclusion Chromatography - Multi-Angle Light Scattering (SEC-MALS)

Size-exclusion chromatography combined with multi-angle light scattering was performed to determine the homogeneity and the native molecular mass of all proteins under study. Analyses were performed on an LC20 Prominence HPLC equipped with a refractive index detector RID-10A and the photodiode array detector SPD-M20A (all from Shimadzu, Japan). In-line MALS was analyzed either with a miniDAWN TREOS II MALS (for analysis of Spike) or a Heleos Dawn8+ plus QELS apparatus (Wyatt Technology, United States). Prior to analysis, all proteins were centrifuged (16 000 g, 10 min, 20°C) and filtered (0.1 µm Ultrafree-MC filter, Merck Millipore, Germany). Proper performance of the MALS detectors was validated with bovine serum albumin. Purified Spike was analyzed by injection of a total of 50 µg onto a Superose 6 Increase 10/300 GL column (Cytiva, United States) at a flow rate of 0.25 mL min⁻¹. The mobile-phase buffer used was PBS supplemented with 10% glycerol (pH 7.4). All other proteins were analyzed by using a Superdex 200 10/300 GL column (Cytiva, United States) equilibrated with PBS plus 200 mM NaCl (pH 7.4). A total of 25 µg of each protein was injected and experiments were performed at a flow rate of 0.75 mL min⁻¹. Data were analyzed using the ASTRA 6 software (Wyatt Technology, United States).

### Example 9: Peptide mapping and glycopeptide analysis

All samples were analyzed as in-solution proteolytic digests of the respective proteins by LC-ESI-MS(/MS). For this, the pH of the samples was adjusted to pH 7.8 by the addition of 1 M HEPES, pH 7.8 to a final concentration of 100 mM. The samples were then chemically reduced and S-alkylated, using a final concentration of 10 mM dithiothreitol for 30 min at 56°C, and a final concentration of 20 mM iodoacetamide for 30 min at room temperature in the dark. To maximize sequence coverage, proteins were digested for 18 h at 37°C with chymotrypsin (Roche), followed by 3 h at 37°C using trypsin (Promega). All proteolytic digests were acidified by addition of 10% formic acid to pH 2 and directly analyzed by LC-ESI-MS(/MS), using a capillary BioBasic C18 reversed-phase column (BioBasic-18, 150 x 0.32 mm, 5 µm, Thermo Scientific), installed in an Ultimate U3000 HPLC system (Dionex), developing a linear gradient from 95% eluent A (80 mM ammonium formate, pH 3.0, in HPLC-grade water) to 65% eluent B (80% acetonitrile in 80 mM ammonium formate, pH 3.0) over 50 min, followed by a linear gradient from 65% to 99% eluent B over 15 min, at a constant flow rate of 6 µL/min, coupled to a maXis 4G Q-TOF instrument (Bruker Daltonics; equipped with the standard ESI source). For (glyco)peptide detection and identification, the mass spectrometer was operated in positive ion DDA mode (i.e. switching to MS/MS mode for eluting peaks), recording MS scans in the m/z range from 150 to 2200 Th, with the 6 highest signals selected for MS/MS fragmentation. Instrument calibration was performed using a commercial ESI calibration mixture (Agilent). Site-specific profiling of protein glycosylation was performed using the dedicated Q-TOF data-analysis software packages Data Analyst (Bruker Daltonics) and Protein Scape (Bruker Daltonics), in conjunction with the MS/MS search engine MASCOT (Matrix Sciences Ltd.) for automated peptide identification.

### Example 10: ACE2 activity assays

Enzymatic activity of ACE2 was determined and quantified as described previously (Vickers et al., J. Biol. Chem. 277, 14838-14843 (2002)), using 100 µM 7-methoxycoumarin-4-yl-acetyl-Ala-Pro-Lys-2,4-dinitrophenyl (Bachem, Bubendorf, Switzerland) as substrate.

### Example 11: SARS-CoV-2 neutralization assays

Vero E6 cells (ATCC) were grown in Dulbecco's Modified Eagle's Medium (DMEM, Thermofisher) supplemented with 1% non-essential amino acids (Thermofisher), 10 mM HEPES (Thermofisher) and 10% FBS at 37°C, 5% CO₂. SARS-CoV-2 isolated from a nasopharyngeal sample of a Swedish COVID-19 patient (GenBank accession number MT093571) was propagated in Vero E6 cells. Virus was titered using a plaque assay as previously described (Becker et al., Proc. Natl. Acad. Sci. USA 105, 19944-19949 (2008)) with fixation of cells 72 hours post infection. Vero E6 cells were treated and infected as described previously (Monteil et al., Cell 181, 905-913.e7 (2020)). Briefly, Vero E6 cells were seeded in 48-well plates (5 × 10⁴ cells per well) in DMEM containing 10% FBS. 24 hours post-seeding, wild type ACE2-Fc, ACE2-Fc glycovariants, rshACE2 and deglycosylated rshACE2 were mixed with different amounts of virus in a 1:1 ratio in a final volume of 100 µl per well in DMEM containing 5% FBS, incubated for 30 min at 37°C and then added to the cells. 15 hours post-infection, cells were washed 3 times with PBS and then lysed using Trizol (Thermofisher) before analysis by RT-qPCR to quantify the content of SARS-CoV-2 RNA.

### Example 12: Statistics

All statistical analyses were conducted using GraphPad Prism 8 (GraphPad). Significance was determined by one-way ANOVA followed by Students t-test for internal groups (*, P<0.05; **, P<0.01; ***, P<0.001).

### Example 13: ACE2-Spike interactions

To assess the impact of all seven individual N-glycosylation sites of human ACE2 on its interaction with Spike, we first elucidated the entire glycome of rshACE2 (Fig. 5) and recombinant trimeric Spike. We then constructed 3D models of complex or oligo-mannosidic glycan trees, and connected these onto the respective sites of ACE2 and Spike (Table 2). Based on the site specific glycosylation profiles we then constructed atomistic models of the trimeric Spike glycoprotein, free dimeric ACE2 and the Spike glycoprotein in complex with ACE2 (Fig. 1A-C, 2A, B). Using these fully glycosylated structures, molecular dynamics simulations of the Spike-ACE2 complex and of free ACE2 were performed. Inspection of the most important interacting residues on Spike and ACE2, their average distances and the electrostatic potential of the interface area identified critical contact sites (Figs. 6 and 7), readily explaining why Spike binds to human but not mouse ACE2. These data provide a useful atomic scale model of the Spike and ACE2 glyco-trees.

Next, the complete solvent accessible surface areas (SASA) of the Spike protein in complex with ACE2, both with and without glycans, were quantified. The average accessible area of protein atoms for non-glycosylated and glycosylated Spike was 1997 nm² and 1420 nm², respectively, indicating that glycans shield about 29% of the protein surface of Spike. The area of protein atoms that are shielded by the individual glycans are shown in Fig. 1D and Fig. 8. The receptor binding domain (RBD) of Spike exists in two distinct conformations, referred to as "up" and "down" (Walls et al., Cell 181, 281-292.e6 (2020)). The "up" conformation corresponds to the receptor-accessible state with the RBD of one monomer exposed (dark green in Figs. 1A-C and 2A, B). Further analysis showed that glycans at N122, N165 and N343 on Spike directly interact with ACE2 or its glycans (Fig 1B, C, E, F). Recently it has been reported that Spike mutants lacking the glycans at N331 and N343 display reduced infectivity (Li et al., Cell 182, 1284-1294.e9 (2020)). In the present dynamic simulations, the glycan at position N343 interacts directly with ACE2, while the glycan at N331 interacts with a neighboring Spike monomer (Fig 1C, E, F; and Fig 9A-D), indicating that the N331 glycosite only indirectly affects the interaction of Spike with ACE2, such as stabilizing the "up" conformation. It is also proposed that elimination of the glycosylation motif at N234 results in increased resistance to neutralizing antibodies, without reducing infectivity of the virus. In our model, the glycan at N234 stabilizes the "up" conformation, and its removal allows the RBD to be shielded more effectively. The Man9 glycan at N234 of Spike partially inserts itself into the vacant space in the core of the trimer that is created when the RBD of monomer 3 is in the "up" conformation (Fig. 10). However, we did not observe this throughout the entire course of our simulation, suggesting that the free space created by the "up" conformation is smaller for Spike in complex with ACE2, and that binding to ACE2 has a marked stabilizing effect on the Spike monomer. The N165Q mutant was experimentally found to be more sensitive to neutralization (Li et al., *supra*). In our models, the glycan at N165 is positioned directly next to the RBD (Fig. 1B) and thus could shield important antigenic sites. These data highlight the complex impact of Spike glycosylation on the intramolecular interactions of the Spike monomers and, critically, the interaction with ACE2, posing a challenge to design SARS-CoV-2 neutralizing moieties.

Since our modeling clearly confirmed that ACE2 glycosylation plays a significant role in its binding to Spike, we also determined the SASA of the Spike-ACE2 interface region. While the total interface area was 23.1 nm², glycans accounted for up to 45% of the interface area, i.e. 10.4 nm², contributed by the four most relevant glycans at positions N53, N90, N322 and N546 of ACE2 (Fig. 2A, B, G).

### Example 14: Specific N-glycan interactions

To identify candidate N-glycans of ACE2 that might influence binding to Spike, we then scored the number of atoms of each ACE2 glycan in contact with Spike. A contact was defined as a distance of < 0.6 nm between two atoms. This allowed us to identify the glycans at N53, N90, N322 and N546 as interacting with Spike, with the glycan at position N53 having the weakest interaction (Fig. 2E). The degree of interaction correlated with the spatial proximity between the glycans and the RBD. However, assessing the numbers of hydrogen bonds that formed during the simulations, the difference between N90, N322, and N546 was less pronounced (Fig. 2F). Interestingly, the glycans at N90 and N322 interacted directly with Spike protein atoms, while the glycan at N546 (red sticks in Fig. 2B) interacts mostly with the glycans at N122 and N165 of Spike (dark green and orange sticks in Fig. 1B). Next, we assessed the conformational freedom of ACE2 glycans upon binding to Spike and compared their respective density maps in the simulations of free ACE2, and ACE2 in complex with Spike. The density maps revealed reduced conformational freedom of ACE2 glycans when in complex with Spike, especially for glycans at N90, N322 and N546 (Fig. 2C, D). These data uncover three critical glycans on ACE2 that we predict to hamper binding to Spike, either sterically or through an entropic penalty upon binding due to a loss of conformational freedom. These three glycans have been implicated as being relevant for binding before (Zhao et al., *supra*), as well as the glycan at N53, but no conclusions were drawn if they contribute positively or negatively to binding. Mehdipour and Hummer predict the glycan at N322 to contribute favourably to binding (Mehdipour et al., bio-Rxiv 2020.07.09.193680 (2020). doi: 10.1101/2020.07.09.193680), while our work rather suggests the opposite effect.

### Example 15: Site specific N-glycan ablation

Since only the glycans at N90 and N322 directly interact with the protein atoms of the Spike proteins, while the glycan on N546 forms hydrogen bonds with glycans present on Spike, we focused our studies on the N90 and N322 glycosylation sites. First, we ablated the N-glycosylation sites at N90 and N322 individually in ACE2-Fc fusion constructs, expressed them in HEK293-6E cells (Figs. 11 and 12) and characterized the binding properties of the purified proteins by biolayer interferometry (BLI). For this, ACE2-wt-Fc, ACE2-T92Q-Fc (Chan et al., Science 369, 1261-1265 (2020)) and ACE2-N322Q-Fc were biotinylated, immobilized on streptavidin biosensor tips and dipped into serial dilutions of trimeric Spike. Since we did not observe appreciable dissociation of ACE2-Fc/trimeric Spike complexes in our analyses (Fig. 3A), we evaluated the association rates (*k*_{obs}; Fig. 3B). Also, equilibrium affinity constants (*K*_{D}) were determined for the interactions between the immobilized ACE2-Fc constructs and monomeric RBD (Fig. 3C, Fig. 13). The BLI data are in agreement with our computational models, confirming that the removal of protein N-glycosylation at either N90 or N322 results in 2 to 3 times higher binding affinities, when compared to ACE2-wt-Fc (ACE2-wt-Fc *K*_{D} = 22.8 nM, ACE2-T92Q-Fc *K*_{D} = 8.0 nM and ACE2-N322Q-Fc *K*_{D} = 11.4 nM; Fig. 3C). Thus, structure-guided glyco-engineering at N90 and N322 results in ACE2 with increased affinity for SARS-CoV-2 Spike binding.

### Example 16: Virus neutralization

Next, we tested the virus neutralization properties of ACE2-wt-Fc, ACE2-T92Q-Fc and ACE2-N322Q-Fc. For this, we infected Vero E6 cells with 10⁶ plaque-forming units (PFU; multiplicity of infection 20) of SARS-CoV-2 in the presence of either ACE2-wt-Fc, ACE2-T92Q-Fc, or ACE2-N322Q-Fc (Fig. 4A,B). The extent of SARS-CoV-2 infection and replication was quantified by RT-qPCR detection of viral RNA extracted from the cells. Untreated SARS-CoV-2 infected cells yielded approximately 10,000 times more viral RNA than ACE2-wt-Fc treated cells. Importantly, co-incubation of cells with SARS-CoV-2 and ACE2-T92Q-Fc or ACE2-N322Q-Fc resulted in significant further reduction of the viral load (9 and 4 times less viral RNA, respectively) when compared to ACE2-wt-Fc (Fig. 4A,B). Thus, in line with our structural glycan interaction map, the removal of protein glycosylation at N90 and N322 gave rise to a highly potent ACE2 decoy receptor, with significantly improved SARS-CoV-2 neutralization properties.

To investigate a potential additive effect of simultaneous elimination of N-glycosylation at N90 and N322, we generated a double mutant ACE2-T92Q-N322Q-Fc construct (Fig. 11 and 12). We also digested ACE2-wt-Fc with peptide-N4-(N-acetyl-beta-glucosaminyl)asparagine amidase F (PNGase F) to remove all accessible N-glycans (deglyco-ACE2-wt-Fc). The complete release of all N-glycans from all sites with the exception of N546 was confirmed by LC-ESI-MS/MS (Fig. 14). The glycans at N546 of ACE2-wt-Fc exhibited partial resistance (40%) to our enzymatic treatment (Fig. 14). Of note, enzymatic removal of N-glycans did not impede the structural stability and homogeneity of ACE2-Fc as assessed by size-exclusion chromatography coupled to multi-angle light scattering (SEC-MALS; Fig. 12B). Measurements of the interaction between ACE2-T92Q-N322Q-Fc and deglyco-ACE2-wt-Fc with RBD by BLI analysis yielded *K*_{D} values similar to those determined for single mutant ACE2-T92Q-Fc (ACE2-T92Q-N322Q-Fc *K*_{D} = 8.2 nM; deglyco-ACE2-wt-Fc *K*_{D} = 7.6 nM; Fig. 3A-C). Moreover, the effects of ACE2-T92Q-N322Q-Fc and deglyco-ACE2-wt-Fc on SARS-CoV-2 infections of Vero E6 cells was comparable to single mutant ACE2-T92Q-Fc (Fig. 4A,B). These data identify critical glycans at position N90 and N322 of ACE2 that structurally and functionally interfere with Spike-ACE2 binding; removal of these glycans via site-directed mutagenesis generated an ACE2 variant with improved binding to the Spike of SARS-CoV-2.

### Example 17: Deglycosylated ACE2

While this data uncovers the critical glycans at the ACE2-Spike interface, these experiments prompted us to test the feasibility of removing all N-glycans from clinical grade rshACE2, which is in phase 2b clinical testing in COVID-19 patients, and to test for its SARS-CoV-2 neutralization properties. To this end, we generated enzymatically deglycosylated clinical grade rshACE2 (deglyco-rshACE2) using PNGase F. The quantitative release of all N-glycans, with the exceptions of those attached to the N432 and N546 glyco-sites, was confirmed by LC-ESI-MS/MS and homogeneity of the dimeric deglyco-rshACE2 was validated by SEC-MALS (Fig. 12 and 14). Paralleling our initial observations with deglyco-ACE2-wt-Fc, we found the binding affinity of deglyco-rshACE2 to RBD (*K*_{D} = 5.1 nM) to be two times higher than for glycosylated rshACE2 (*K*_{D} = 10.5 nM; Fig. 3A-C). Furthermore, deglyco-rshACE2 displayed improved SARS-CoV-2 neutralization properties in Vero E6 cell infection assays, where we observed a 5-fold reduction in intracellular SARS-CoV-2 viral load when compared to treatment with native rshACE2 (Fig. 4C).

### Example 18: Enzymatic activity of ACE2

Besides serving as a soluble decoy receptor to SARS-CoV-2, rshACE2 also regulates blood pressure and protects multiple organs such as the heart, kidney, blood vessels and lung via enzymatic degradation of angiotensin II (Vickers et al., *supra*). In contrast to other recently described ACE2 mutants displaying improved Spike binding but apparently impaired ACE2 enzymatic activity (Chan et al., Science 369, 1261-1265 (2020)), the catalytic activities of ACE2-T92Q-Fc, ACE2-N322Q-Fc and ACE2-T92Q-N322Q-Fc were found to be only slightly reduced as compared to ACE2-wt-Fc (to 53%, 76% and 67%, respectively; Fig. 4D). Interestingly, deglyco-ACE2-wt-Fc exhibited even enhanced enzymatic activity as compared to native ACE2-wt-Fc (147%; Fig. 4E). This was also observed for deglyco-rshACE2, albeit to a lesser extent (115% of rshACE2; Fig. 4E). These results show that enzymatic removal of glycans from ACE2-Fc and clinical grade rshACE2 results in increased Spike binding, enhanced SARS-CoV-2 neutralization, while preserving its critical enzymatic activity.

Taken together, our data provides ACE2 variants with improved SARS-CoV-2 neutralization properties without compromising the catalytic activity of the enzyme. Given the importance of N-glycosylation for proper protein folding and the potential of unwanted immunological side effects when non-natural mutations are introduced into a therapeutic glycoprotein, enzymatic deglycosylation of native rshACE2 offers an industrially feasible approach to generate a next-generation drug for COVID-19. Our *in silico* models of the Spike-ACE2 interaction led to predictions that were confirmed in *in vitro* binding studies and cell-based neutralization assays. Engineering of ACE2 N-glycosylation by site-directed mutagenesis resulted in enhanced binding affinities and improved virus neutralization. Importantly, removal of all N-glycans from clinical grade rshACE2 yields a superior SARS-CoV-2 decoy receptor with high promise as effective treatment for COVID-19 patients. Deglycosylated rshACE2 shows superior therapeutic properties for the treatment of severe COVID-19 cases.

## Claims

1. An ACE2 polypeptide lacking an N-linked glycosylation of two or more sugar residues at an amino acid corresponding to Asn90 and/or Asn322 of SEQ ID NO: 1 or comprising a truncated N-linked glycosylation at an amino acid corresponding to Asn90 and/or Asn322 of SEQ ID NO: 1, wherein the truncated N-linked glycosylation is not larger than a -GlcNAc₂Man₃GlcNAc₂ structure, and, optionally, comprising an N-linked glycosylation of two or more sugar residues at one or more Asn residues corresponding to Asn53, Asn103, Asn432, Asn546 and/or Asn690 of SEQ ID NO: 1.

2. An ACE2 polypeptide lacking an N-linked glycosylation of two or more sugar residues at an amino acid corresponding to Asn53, Asn90, Asn103, Asn322, Asn432, Asn546 and/or Asn690 of SEQ ID NO: 1 or having a truncated N-linked glycosylation at an amino acid corresponding to Asn53, Asn90, Asn103, Asn322, Asn432, Asn546 and/or Asn690 of SEQ ID NO: 1, wherein the truncated N-linked glycosylation is not larger than a -GlcNAc₂Man₃GlcNAc₂ structure, further comprising at least one immunoglobulin domain.

3. An ACE2 polypeptide obtainable by a) expressing an ACE2 polypeptide in a mammalian cell and b) contacting said ACE2 polypeptide with a beta-N-acetylglucosaminidase such as peptide-N4-(N-acetyl-beta-glucosaminyl)asparagine amidase F (PNGase F).

4. The ACE2 polypeptide according to any of claims 1 to 3, wherein the ACE2 polypeptide lacks an N-linked glycosylation of two or more sugar residues at amino acids corresponding to Asn53, Asn90, Asn103, Asn322, Asn432 and Asn546 of SEQ ID NO: 1 or has a truncated N-linked glycosylation at an amino acid corresponding to Asn53, Asn90, Asn103, Asn322, Asn432 and Asn546 of SEQ ID NO: 1.

5. The ACE2 polypeptide according to any of claims 1 to 4, wherein the ACE2 polypeptide contains a mutation in the N-linked glycosylation sequence corresponding to Asn90-Leu91-Thr92 of SEQ ID NO:1 and/or in the N-linked glycosylation sequence corresponding to Asn322-Met323-Thr324, wherein said mutation removes the N-linked glycosylation consensus sequence Asn-X-Ser/Thr, where X is any amino acid except proline, at amino acids corresponding to amino acids 90-92 and/or 322-324 of SEQ ID NO:1.

6. The ACE2 polypeptide of any of claims 1-5 that is a fusion protein comprising a heavy chain immunoglobulin domain, preferably an antibody CH1, CH2 or CH3 domain, more preferably comprising an Fc fragment.

7. An ACE2 polypeptide lacking an N-linked glycosylation of two or more sugar residues at an amino acid corresponding to Asn53, Asn90, Asn103, Asn322, Asn432, Asn546 and/or Asn690 of SEQ ID NO: 1 or having a truncated N-linked glycosylation at an amino acid corresponding to Asn53, Asn90, Asn103, Asn322, Asn432, Asn546 and/or Asn690 of SEQ ID NO: 1, wherein the truncated N-linked glycosylation is not larger than a -GlcNAc₂Man₃GlcNAc₂ structure, for use as a medicament, wherein the ACE2 polypeptide optionally is an ACE2 polypeptide of any of claims 1-6.

8. The ACE2 polypeptide of any one of claims 1 to 6 or the ACE2 polypeptide for use according to claim 7 lacking N-linked glycosylation of two or more sugar residues at an amino acid corresponding to Asn90 and/or Asn322 of SEQ ID NO: 1.

9. The ACE2 polypeptide of any one of claims 1 to 6 or the ACE2 polypeptide for use according to any of claims 7 or 8, wherein the ACE2 polypeptide is a soluble ACE2 polypeptide, preferably wherein the ACE2 polypeptide lacks the transmembrane domain of ACE2.

10. The ACE2 polypeptide of any one of claims 1 to 6 or 9 or the ACE2 polypeptide for use according to any of claims 7 to 9, wherein the ACE2 polypeptide comprises amino acids corresponding to amino acids 19 to 600 of SEQ ID NO: 1 or having at least 90% sequence identity thereto.

11. The ACE2 polypeptide of any one of claims 1 to 6 or 9 or the ACE2 polypeptide for use according to any of claims 7 to 9, wherein the ACE2 polypeptide comprises amino acids corresponding to amino acids 18 to 740 of SEQ ID NO: 1 or having at least 90% sequence identity thereto.

12. An ACE2 polypeptide lacking an N-linked glycosylation of one or more sugar residues at an amino acid corresponding to Asn53, Asn90, Asn103, Asn322, Asn432, Asn546 and/or Asn690 of SEQ ID NO: 1 or having a truncated N-linked glycosylation at an amino acid corresponding to Asn53, Asn90, Asn103, Asn322, Asn432, Asn546 and/or Asn690 of SEQ ID NO: 1, wherein the truncated N-linked glycosylation is not larger than a -GlcNAc₂Man₃GlcNAc₂ structure, for use in treating or preventing a coronavirus infection, preferably a SARS-CoV-2 infection, in a subject, wherein the ACE2 polypeptide optionally is the ACE2 polypeptide of any of claims 1 to 6 or 9 to 11 or the ACE2 polypeptide for use according to any of claims 7 to 11.

13. The ACE2 polypeptide for use according to claim 12, wherein the ACE2 polypeptide is administered at a dose of 10 µg/kg to 1500 µg/kg daily.

14. A method for treating a coronavirus infection, preferably a SARS-CoV-2 infection, in a subject comprising administering an ACE2 polypeptide to said subject, wherein the ACE2 polypeptide lacks an N-linked glycosylation of one or more sugar residues at an amino acid corresponding to Asn53, Asn90, Asn103, Asn322, Asn432, Asn546 and/or Asn690 of SEQ ID NO: 1 or has a truncated N-linked glycosylation at an amino acid corresponding to Asn53, Asn90, Asn103, Asn322, Asn432, Asn546 and/or Asn690 of SEQ ID NO: 1, wherein the truncated N-linked glycosylation is not larger than a -GlcNAc₂Man₃GlcNAc₂ structure,
wherein the ACE2 polypeptide optionally is the ACE2 polypeptide of any of claims 1 to 6 or 9 to 11 or the ACE2 polypeptide for use according to any of claims 7 to 13.

15. A composition comprising at least 700 µg ACE2 polypeptides, wherein at least 20% (molar-%) of the ACE2 polypeptides are the ACE2 polypeptides as defined in any one of claims 1 to 14; or a composition comprising at least 160 µg ACE2 polypeptides as defined in any one of claims 1 to 14.

16. A nucleic acid encoding an ACE2 polypeptide of any of claims 5 to 6, or a host cell comprising said nucleic acid.

17. An *in vitro* method of manufacturing an ACE2 polypeptide according to any of claims 1 to 6 or a composition according to claim 15, comprising expressing a nucleic acid encoding the ACE2 polypeptide in one or more cells, and, optionally, deglycosylating said ACE2 polypeptide with a beta-N-acetylglucosaminidase such as peptide-N4-(N-acetyl-beta-glucosaminyl)asparagine amidase F (PNGase F).


SEQUENCE LISTING

<110> IMBA Institut für Molekulare Biotechnologie GmbH

<120> Superior neutralization of SARS-CoV-2 by deglycosylated human
angiotensin converting enzyme 2

<130> 12961 P 7930 EP

<160> 2

<170> BiSSAP 1.3.6

<210> 1
<211> 740
<212> PRT
<213> Homo sapiens

<220>
<221> SIGNAL
<222> 1..17
<223> Signal sequence of ACE2

<220>
<223> Truncated human ACE2 (amino acids 1-740)

<220>
<221> DOMAIN
<222> 19..740
<223> Extracellular domain of ACE2

<220>
<221> CARBOHYD
<222> 53
<223> Potential glycosylation site

<220>
<221> CARBOHYD
<222> 90
<223> Potential glycosylation site

<220>
<221> CARBOHYD
<222> 103
<223> Potential glycosylation site

<220>
<221> CARBOHYD
<222> 322
<223> Potential glycosylation site

<220>
<221> CARBOHYD
<222> 432
<223> Potential glycosylation site

<220>
<221> CARBOHYD
<222> 546
<223> Potential glycosylation site

<220>

<221> CARBOHYD
<222> 690
<223> Potential glycosylation site

<400> 1
Met Ser Ser Ser Ser Trp Leu Leu Leu Ser Leu Val Ala Val Thr Ala
1               5                   10                  15
Ala Gln Ser Thr Ile Glu Glu Gln Ala Lys Thr Phe Leu Asp Lys Phe
            20                  25                  30
Asn His Glu Ala Glu Asp Leu Phe Tyr Gln Ser Ser Leu Ala Ser Trp
        35                  40                  45
Asn Tyr Asn Thr Asn Ile Thr Glu Glu Asn Val Gln Asn Met Asn Asn
    50                  55                  60
Ala Gly Asp Lys Trp Ser Ala Phe Leu Lys Glu Gln Ser Thr Leu Ala
65                  70                  75                  80
Gln Met Tyr Pro Leu Gln Glu Ile Gln Asn Leu Thr Val Lys Leu Gln
                85                  90                  95
Leu Gln Ala Leu Gln Gln Asn Gly Ser Ser Val Leu Ser Glu Asp Lys
            100                 105                 110
Ser Lys Arg Leu Asn Thr Ile Leu Asn Thr Met Ser Thr Ile Tyr Ser
        115                 120                 125
Thr Gly Lys Val Cys Asn Pro Asp Asn Pro Gln Glu Cys Leu Leu Leu
    130                 135                 140
Glu Pro Gly Leu Asn Glu Ile Met Ala Asn Ser Leu Asp Tyr Asn Glu
145                 150                 155                 160
Arg Leu Trp Ala Trp Glu Ser Trp Arg Ser Glu Val Gly Lys Gln Leu
                165                 170                 175
Arg Pro Leu Tyr Glu Glu Tyr Val Val Leu Lys Asn Glu Met Ala Arg
            180                 185                 190
Ala Asn His Tyr Glu Asp Tyr Gly Asp Tyr Trp Arg Gly Asp Tyr Glu
        195                 200                 205
Val Asn Gly Val Asp Gly Tyr Asp Tyr Ser Arg Gly Gln Leu Ile Glu
    210                 215                 220
Asp Val Glu His Thr Phe Glu Glu Ile Lys Pro Leu Tyr Glu His Leu
225                 230                 235                 240
His Ala Tyr Val Arg Ala Lys Leu Met Asn Ala Tyr Pro Ser Tyr Ile
                245                 250                 255
Ser Pro Ile Gly Cys Leu Pro Ala His Leu Leu Gly Asp Met Trp Gly
            260                 265                 270
Arg Phe Trp Thr Asn Leu Tyr Ser Leu Thr Val Pro Phe Gly Gln Lys
        275                 280                 285
Pro Asn Ile Asp Val Thr Asp Ala Met Val Asp Gln Ala Trp Asp Ala
    290                 295                 300
Gln Arg Ile Phe Lys Glu Ala Glu Lys Phe Phe Val Ser Val Gly Leu
305                 310                 315                 320
Pro Asn Met Thr Gln Gly Phe Trp Glu Asn Ser Met Leu Thr Asp Pro
                325                 330                 335
Gly Asn Val Gln Lys Ala Val Cys His Pro Thr Ala Trp Asp Leu Gly
            340                 345                 350
Lys Gly Asp Phe Arg Ile Leu Met Cys Thr Lys Val Thr Met Asp Asp
        355                 360                 365
Phe Leu Thr Ala His His Glu Met Gly His Ile Gln Tyr Asp Met Ala
    370                 375                 380
Tyr Ala Ala Gln Pro Phe Leu Leu Arg Asn Gly Ala Asn Glu Gly Phe
385                 390                 395                 400
His Glu Ala Val Gly Glu Ile Met Ser Leu Ser Ala Ala Thr Pro Lys
                405                 410                 415
His Leu Lys Ser Ile Gly Leu Leu Ser Pro Asp Phe Gln Glu Asp Asn
            420                 425                 430
Glu Thr Glu Ile Asn Phe Leu Leu Lys Gln Ala Leu Thr Ile Val Gly
        435                 440                 445
Thr Leu Pro Phe Thr Tyr Met Leu Glu Lys Trp Arg Trp Met Val Phe
    450                 455                 460

Lys Gly Glu Ile Pro Lys Asp Gln Trp Met Lys Lys Trp Trp Glu Met
465                 470                 475                 480
Lys Arg Glu Ile Val Gly Val Val Glu Pro Val Pro His Asp Glu Thr
                485                 490                 495
Tyr Cys Asp Pro Ala Ser Leu Phe His Val Ser Asn Asp Tyr Ser Phe
            500                 505                 510
Ile Arg Tyr Tyr Thr Arg Thr Leu Tyr Gln Phe Gln Phe Gln Glu Ala
        515                 520                 525
Leu Cys Gln Ala Ala Lys His Glu Gly Pro Leu His Lys Cys Asp Ile
    530                 535                 540
Ser Asn Ser Thr Glu Ala Gly Gln Lys Leu Phe Asn Met Leu Arg Leu
545                 550                 555                 560
Gly Lys Ser Glu Pro Trp Thr Leu Ala Leu Glu Asn Val Val Gly Ala
                565                 570                 575
Lys Asn Met Asn Val Arg Pro Leu Leu Asn Tyr Phe Glu Pro Leu Phe
            580                 585                 590
Thr Trp Leu Lys Asp Gln Asn Lys Asn Ser Phe Val Gly Trp Ser Thr
        595                 600                 605
Asp Trp Ser Pro Tyr Ala Asp Gln Ser Ile Lys Val Arg Ile Ser Leu
    610                 615                 620
Lys Ser Ala Leu Gly Asp Lys Ala Tyr Glu Trp Asn Asp Asn Glu Met
625                 630                 635                 640
Tyr Leu Phe Arg Ser Ser Val Ala Tyr Ala Met Arg Gln Tyr Phe Leu
                645                 650                 655
Lys Val Lys Asn Gln Met Ile Leu Phe Gly Glu Glu Asp Val Arg Val
            660                 665                 670
Ala Asn Leu Lys Pro Arg Ile Ser Phe Asn Phe Phe Val Thr Ala Pro
        675                 680                 685
Lys Asn Val Ser Asp Ile Ile Pro Arg Thr Glu Val Glu Lys Ala Ile
    690                 695                 700
Arg Met Ser Arg Ser Arg Ile Asn Asp Ala Phe Arg Leu Asn Asp Asn
705                 710                 715                 720
Ser Leu Glu Phe Leu Gly Ile Gln Pro Thr Leu Gly Pro Pro Asn Gln
                725                 730                 735
Pro Pro Val Ser
            740

<210> 2
<211> 805
<212> PRT
<213> Homo sapiens



<220>
<221> SIGNAL
<222> 1..17
<223> Signal sequence of ACE2

<220>
<223> Full-length amino acid sequence of human ACE2

<220>
<221> DOMAIN
<222> 19..740
<223> Extracellular domain of ACE2

<220>
<221> CARBOHYD
<222> 53
<223> Potential glycosylation site

<220>
<221> CARBOHYD

<222> 90
<223> Potential glycosylation site

<220>
<221> CARBOHYD
<222> 103
<223> Potential glycosylation site

<220>
<221> CARBOHYD
<222> 322
<223> Potential glycosylation site

<220>
<221> CARBOHYD
<222> 432
<223> Potential glycosylation site

<220>
<221> CARBOHYD
<222> 546
<223> Potential glycosylation site

<220>
<221> CARBOHYD
<222> 690
<223> Potential glycosylation site

<220>
<221> DOMAIN
<222> 741..761
<223> Transmembrane domain of ACE2

<220>
<221> DOMAIN
<222> 762..805
<223> Cytoplasmic domain of ACE2

<400> 2